# EUROPEAN PATENT APPLICATION

(11) **EP 3 254 701 A1**
(43) Date of publication of application: **13.12.2017**
(21) Application number: 16173740.8
(22) Date of filing: 09.06.2016
(51) Int. Cl.: A61K 47/64

(54) **CBL-B INHIBITOR COMPRISING A CBL-B BINDING PEPTIDE FOR THE PREVENTION OR TREATMENT OF FUNGAL INFECTIONS**

(71) Applicant: IMBA-Institut für Molekulare Biotechnologie GmbH, 1030 Wien (AT)
(72) Inventor: The designation of the inventor has not yet been filed
(74) Representative: Sonn & Partner Patentanwälte

(57) **Abstract**

The present invention relates to the prevention or treatment of fungal infections. The present invention provides a Cbl-b inhibitor for use in the prevention or treatment of a fungal infection in a patient, a Cbl-b inhibitor comprising a binding peptide of Syk, a fusion peptide comprising a cell delivery portion and a Cbl-b binding peptide, wherein the Cbl-b binding peptide binds to the TKB domain of Cbl-b, kits and uses for the same.

## Description

### Field of the invention

The present invention relates to novel Cbl-b inhibitors and the inhibition of Cbl-b to modulate the activity of the immune system, especially in the prevention or treatment of fungal infections, in particular candidiasis, or cancer.

### Background of the invention

The E3-ubiquitin-ligase Cbl-b (casitas b-lineage lymphoma-b) has originally been described as a negative regulator of the adaptive immune response. Both T cell receptor (TCR) signaling and B cell receptor (BCR) signaling are modulated by Cbl-b mediated ubiquitination of critical signaling molecules. Consequently Cbl-b has been a target for immune therapy in the treatment of several diseases, especially cancer (US 2010/0260808 A). US 8,168,176 B2 describes methods of treating endotoxin-mediated disorders, including sepsis by administering Cbl-b. Additional roles for Cbl-b are in NK cell-mediated anti-tumor immunity (US 2014/0010781 A), FcεR signaling in mast cells, integrin signaling, and DC functions, extending the range of Cbl-b functions to cells of the innate immune system. Signaling molecules targeted by Cbl-b are often not specific to defined pathways, but can be used by multiple signaling modules.

Fungi are increasingly recognized as major pathogens in critically ill patients (Enoch et al., 2006) and fungal infections claim an estimated 1.5 million lives every year (Brown et al., 2012). *Candida* species and *Cryptococcus* species are the yeasts most frequently isolated in clinical practice. *Candida albicans* is the most frequent fungal pathogen isolated from patients suffering from severe forms of fungal infections in the developed world (Kullberg et al., 2015). *Aspergillus* species are the most commonly isolated invasive molds (Denning, 1998). The use of antineoplastic and immunosuppressive agents, broad-spectrum antibiotics, prosthetic devices and grafts, and more aggressive surgery are reasons for the increase in invasive fungal infections. Patients with burns or pancreatitis are also predisposed to fungal infection (Enoch et al., 2006). Patients with an impaired immune status, as observed upon HIV infection (Armstrong-James et al., 2014; Brown et al., 2014; Park et al., 2009), immune suppression (Ravikumar et al., 2015), or certain types of primary immune deficiencies (Lanternier et al., 2013; Lortholary et al., 1997) are at high risk of developing a severe fungal infection.

Yeasts, molds and dimorphs are three types of fungi known to cause disease in humans (Jain et al., 2010). Fungal infections are classified into 3 groups: 1) superficial/cutaneous infections, present on skin, hair and nails, 2) subcutaneous infections, present in tissues under the skin and 3) systemic infections.

Several anti-fungal agents are used for the treatment of the different kinds of fungal infections. Amphotericin B, a polyene with a very broad spectrum of activity, including most yeasts and molds, belongs to the class of polyene anti-fungals and can be administered locally or systemically. However, side effects are occurring in 50-90 % of cases, and are usually nephrotoxicity or infusion-related. Fever, chills, nausea, vomiting and hypotension are further side effects. Fluconazole belongs to the class of azole anti-fungals and is administered orally or intravenously. Disadvantages include resistance of several *Candida* strains and adverse effects such as headache, nausea, vomiting and elevated liver enzymes.

Taken together, efficacy and/or safety of known anti-fungal agents are still lacking in many circumstances and there is a significant need for new anti-fungal therapies. It is one of the objects of the present invention to provide novel compounds and methods for preventing or treating fungal infections.

### Summary of the invention

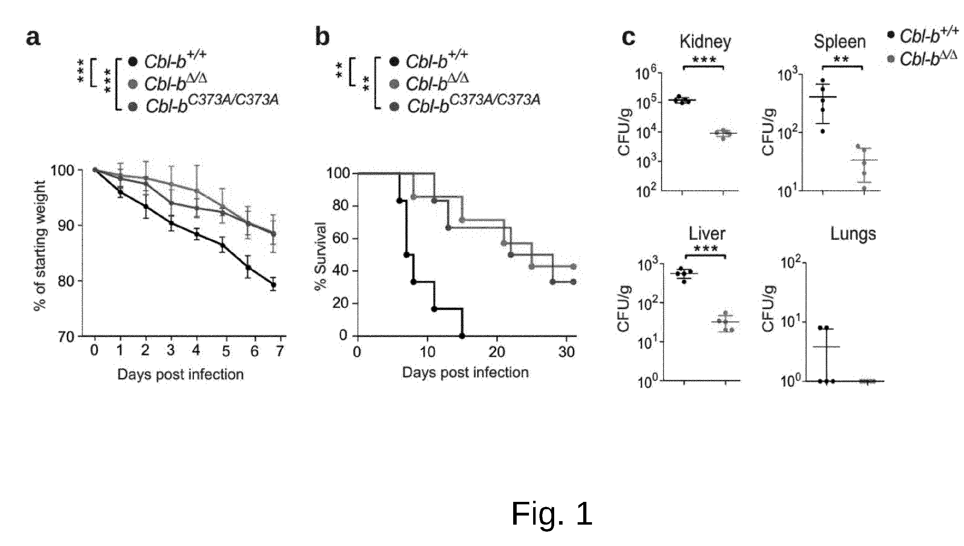

The present invention provides methods and means for inhibition of casitas b-lineage lymphoma-b (Cbl-b) activity, which can significantly ameliorate patients health when diseased or infected, in particular by fungal infections, most strikingly by significantly reducing morbidity (see e.g. Fig. 1a) and mortality (Fig. 1b). Accordingly, the present invention provides a Cbl-b inhibitor for use in the prevention or treatment of a fungal infection in a patient. Preferably, the fungal infection is a yeast infection, more preferably an opportunistic yeast infection. Even more preferably, the fungal infection is a candidiasis or a cryptococcosis. In an embodiment, the Cbl-b inhibitor comprises a binding peptide of spleen tyrosine kinase (Syk).

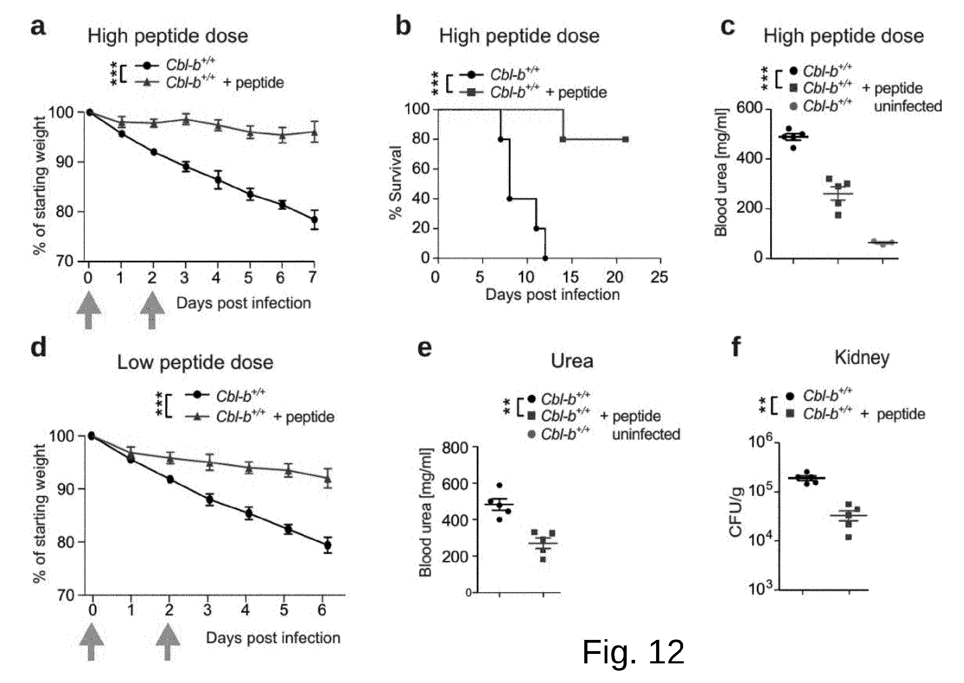

In another aspect of the invention, a fusion peptide comprising a cell delivery portion and a Cbl-b binding peptide, wherein the Cbl-b binding peptide binds to the tyrosine kinase binding (TKB) domain of Cbl-b, is provided. Notably, this fusion peptide has turned out to be very effective in protecting from lethal fungal infections (Fig. 12b), but it can be used as Cbl-b inhibitor in any application or use, including for use in therapy.

Moreover, the invention relates to a fusion peptide comprising a cell delivery portion and a Cbl-b binding peptide, wherein the Cbl-b binding peptide is an intramer or aptamer, and wherein the Cbl-b binding peptide inhibits Cbl-b. Preferably, the Cbl-b binding peptide binds to the TKB domain of Cbl-b.

The present invention further relates to a Cbl-b inhibitor for use in a patient as a vaccine adjuvant, wherein the Cbl-b inhibitor is a Cbl-b binding peptide. Preferably, the Cbl-b binding peptide binds to the TKB domain of Cbl-b. Especially preferred it is a peptide (fragment) of or derived from Syk.

The present invention also relates to a pharmaceutical composition, comprising the fusion peptide or the Cbl-b binding peptide, and a pharmaceutically acceptable carrier promoting cellular uptake of said peptide.

Even further, the invention provides a vaccine, comprising as an adjuvant a Cbl-b inhibitor. Preferably, the Cbl-b inhibitor is a Cbl-b binding peptide of Syk, and at least one antigen.

In another aspect, the present invention also relates to a kit comprising said peptide and a pharmaceutically acceptable excipient. Preferably, the pharmaceutically acceptable excipient is suitable for intracellular administration in the patient.

The present invention also relates to a method for regulating innate immunity, comprising the inhibition of Cbl-b. Preferably, said fusion peptide or Cbl-b inhibitor are administered to the patient.

Moreover, in another aspect, the present invention relates to an *in vitro* or *in vivo* method for stimulating or activating an immune cell, comprising contacting or treating the cell with said fusion peptide or Cbl-b binding peptide.

All aspects and embodiments of the invention relate to the inhibition of Cbl-b using a Cbl-b binding peptide. All general and specific descriptions provided herein in more detail, including preferred embodiments relate to all aspect and uses of the invention equally, except where explicitly stated. E.g. any preferred definition of the peptide or its Cbl-b binding portion can be used in any aspect or method of the invention.

### Detailed description of the invention

The E3-ubiquitin-ligase Cbl-b is a member of the Cbl family of proteins, playing key roles in regulating signaling in a variety of cellular systems (Sohn et al., 2003). The amino-terminal domain of Cbl proteins is composed of a tyrosine kinase binding (TKB) domain and a RING finger domain, which are both modulating signal transduction and protein degradation (Ohno et al., 2016; Peschard et al., 2004). Substrate recognition by Cbl-b is mainly mediated by its TKB domain, which recognizes phosphotyrosines on target molecules, such as ZAP-70, Syk and Src and with residues located in RTKs such as EGFR, Met and CSF-1 receptors (Mancini et al., 2002; Peschard et al., 2001; Peschard et al., 2004; Tsygankov et al., 2001). Cbl-b has originally been characterized as a negative regulator of the adaptive immune response (Bachmaier et al., 2000; Chiang et al., 2000). Both T cell receptor (TCR) signaling and B cell receptor (BCR) signaling are modulated by Cbl-b mediated ubiquitination of critical signaling molecules (Loeser et al., 2007; Kitaura et al., 2007). Genetic ablation of Cbl-b (Bachmaier et al., 2000; Chiang et al., 2000) or knock-in of a catalytically inactive Cb1-b^{c373A/c373A} variant (Paolino et al., 2011) has thus been reported to cause broad autoimmune manifestations, particularly after antigenic challenge. Recent observations uncovered additional roles for Cbl-b in NK cell-mediated anti-tumor immunity (Paolino et al., 2014), FcεR signaling in mast cells (Gustin et al., 2006; Qu et al., 2004), integrin signaling (Han et al., 2010), and DC functions (Wallner et al., 2013), extending the range of Cbl-b functions to cells of the innate immune system. Signaling molecules targeted by Cbl-b are often not specific to defined pathways, but can be used by multiple signaling modules.

Spleen tyrosine kinase (Syk) is a signaling protein targeted by Cbl-b. Canonical signaling induced by, amongst others, the C-type lectin receptors (CLRs) Dectin-1, -2, and -3 is critically dependent on activation of Syk (Gross et al., 2009; Mocsai et al., 2010). Syk is recruited to Dectin-1, Dectin-2/3, and Mincle receptor complexes and subsequently activated by both phosphorylation via Src family kinases and auto-phosphorylation (Deng et al., 2015). Activated Syk then triggers the inflammasome (Gross et al., 2009; Gringhuis et al., 2012; Zwolanek et al., 2014), canonical NF-κB signaling (LeibundGut-Landmann et al., 2007; Ruland, 2008), and production of reactive oxygen species (ROS) in monocytes, macrophages, neutrophils and dendritic cells (DCs) (Mocsai et al., 2010). Collectively, the activation of Syk by CLRs causes an immediate activation of fungicidal pathways, as well as inducing cell type specific transcriptional changes tailored to eradicating local or systemic fungal infections.

According to the invention, it was found that administration of a Cbl-b inhibitor protected from morbidity and mortality induced by infection with *Candida albicans,* the most prevalent yeast pathogen, and similar fungal pathogens. Thus, in a first aspect, the present invention provides a Cbl-b inhibitor for use in the prevention or treatment of a fungal infection in a patient, wherein the fungal infection is a yeast infection. The patient may be in need of a therapy, e.g. having disease symptoms or an adverse prognosis.

The Cbl-b gene and its gene products are well described in the art (see for instance also UniGene Id. Mm.328206 and Hs.430589). Cbl-b sequences are published e.g. in GenBank database under acc. no. NP_001028410.1 (Mus musculus) and NP_733762.2 (Homo sapiens). Cbl-b inhibitors are known in the art. Anti-Cbl-b antibodies, siRNAs and antisense inhibitors suitable as Cbl-b inhibitors are commercially available. Specifically, siRNAs suitable for reduction or inhibition of Cbl-b expression and thus also of Cbl-b function are disclosed for example in US 2007/0054355 A1, US 2010/0260808 A and US 2014/0010781 A (all incorporated herein by reference).

Any Cbl-b inhibitor can be used according to the present invention. In one embodiment, the Cbl-b inhibitor is selected from nucleic acids targeting Cbl-b, such as Cbl-b inhibiting nucleic acids, siRNA, shRNA, miRNA.

It has been shown that the immune response to several clinically relevant yeast infections such as *Candida* infections of the family *Saccharomycetaceae* are regulated by Cbl-b. Thus, in a preferred embodiment, the yeast infection is of a yeast of the family *Saccharomycetaceae,* more preferably a candidiasis or a cryptococcosis. More preferably, the fungal infection is a *Candida albicans, Candida glabrata, Candida krusei* or a *Candida dubliniensis* infection.

The present invention also relates to a Cbl-b inhibitor for use in the prevention or treatment of a fungal infection in a patient, wherein the Cbl-b inhibitor is a Cbl-b binding peptide of Syk. The Syk gene and its gene products are well known in the art (UniGene Id. Mm.375031, Rn.87407, Mmu.32294 and Hs.371720). Syk sequences are published for example in GeneBank database under acc. nr. NP_001185906.2 (Mus musculus), NP_036890.1 (Rattus norvegicus), NM_001261035.1 (Macaca mulatta) and NP_003168.2 (Homo sapiens). Fragments of Syk that contain the sequence responsible for binding to Cbl-b (Cbl-b binding peptide) have been shown to be a particularly effective treatment agent for the therapeutic inhibition of Cbl-b. In particular, the Cbl-b-binding peptide binds to the TKB domain of Cbl-b (a TBK (domain) binding peptide).

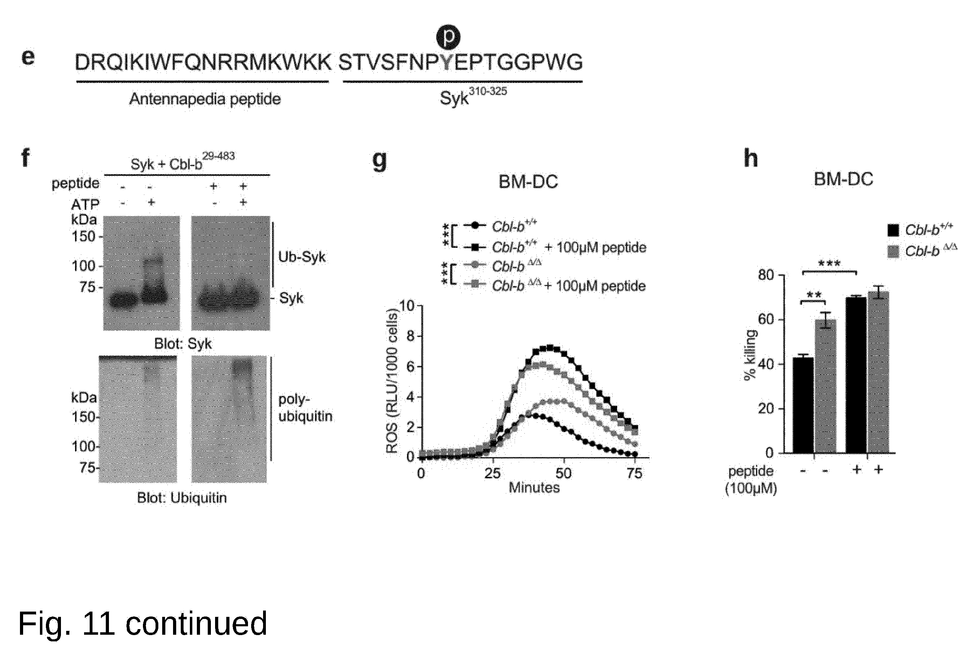

In a preference, the Cbl-b binding peptide comprises the phosphotyrosine and surrounding flanking regions of Syk (Fig. 11e, right part, showing a murine Syk peptide). In one embodiment of the invention, the TKB domain binding peptide has the following consensus sequence: SFNPYEPX₁X₂X₃ (SEQ ID NO: 1), wherein X₁, X₂ and X₃ are selected independently from any amino acid. The amino acids are preferably selected from proteinogenic amino acids, e.g. the peptide may comprise at least 50% proteinogenic amino acids. Also preferred is the inclusion of one or more non-proteinogenic amino acid, especially to modify stability and/or pharmacodynamics of the peptide *in vivo.* The Y (tyrosine) in this sequence is preferably phosphorylated. In mice, the homologous sequence is SFNPYEPTGG (SEQ ID NO: 2) having amino acids 313-322 of murine Syk (Syk³¹³⁻³²²). In rats, the homologous sequence is SFNPYEPTGG (SEQ ID NO: 2) in position 313-322 of Syk (Syk³¹³⁻³²²). In rhesus monkeys, the homologous sequence is SFNPYEPEVA (SEQ ID NO: 3) in position 319-328 of Syk (Syk³¹⁹⁻³²⁸). In humans, the homologous sequence is SFNPYEPELA (SEQ ID NO: 4) in position 296-305 of Syk (Syk²⁹⁶⁻³⁰⁵). Thus, in an especially preferred embodiment, the Cbl-b binding peptide comprises the sequence SFNPYEPTGG (SEQ ID NO: 2) or SFNPYEPEVA (SEQ ID NO: 3) or the sequence SFNPYEPELA (SEQ ID NO: 4). Preferably X₁ is T or E. Preferably X₂ is G, L or V. Preferably, X₃ is G, or A. Also variants of these sequences are possible, therefore, the invention also relates to providing or using peptides comprising a sequence selected from SFNPYEPX₁X₂X₃ (SEQ ID NO: 1), SFNPYEPTGG (SEQ ID NO: 2), SFNPYEPEVA (SEQ ID NO: 3) or SFNPYEPELA (SEQ ID NO: 4) with one or two amino acid deletions or substitutions at any position.

In an especially preferred embodiment of the invention, the Cbl-b binding peptide comprises the following consensus sequence: STVSFNPYEPX₁X₂X₃X₄WX₅ (SEQ ID NO: 5), wherein X₁, X₂, X₃, X₄ and X₅ are selected independently from any amino acid, preferably containing proteinogenic and/or non-proteinogenic amino acids as described above. In mice, the homologous sequence is STVSFNPYEPTGGPWG (SEQ ID NO: 6) in position 310-325 of Syk (Syk³¹⁰⁻³²⁵). In rats, the homologous sequence is STVSFNPYEPTGGAWG (SEQ ID NO: 7) in position 310-325 of Syk (Syk³¹⁰⁻³²⁵). In rhesus monkeys, the homologous sequence is STVSFNPYEPEVAPWA (SEQ ID NO: 8) in position 316-331 of Syk (Syk³¹⁶⁻³³¹). In humans, the homologous sequence is STVSFNPYEPELAPWA (SEQ ID NO: 9) in position 293-308 of Syk (Syk²⁹³⁻³⁰⁸). Thus, in a related embodiment, the Cbl-b binding peptide comprises, preferably consists of, the sequence STVSFNPYEPTGGPWG (SEQ ID NO: 6), STVSFNPYEPTGGAWG (SEQ ID NO: 7), STVSFNPYEPEVAPWA (SEQ ID NO: 8) or STVSFNPYEPELAPWA (SEQ ID NO: 9). Especially, the tyrosine (y) in the indicated sequence portion -PYE- in the disclosed sequences is phosphorylated. Preferably X₁ is T or E. Preferably X₂ is G, L or V. Preferably, X₃ is G, or A. Preferably, X₄ is P or A. Preferably, X₅ is G or A. Also variants of these sequences are possible, therefore, the invention also relates to providing or using peptides comprising a sequence selected from STVSFNPYEPX₁X₂X₃X₄WX₅ (SEQ ID NO: 5), STVSFNPYEPTGGPWG (SEQ ID NO: 6), STVSFNPYEPTGGAWG (SEQ ID NO: 7), STVSFNPYEPEVAPWA (SEQ ID NO: 8) or STVSFNPYEPELAPWA (SEQ ID NO: 9) with one, two or three amino acid deletions or substitutions at any position.

Preferably the Cbl-b binding peptide (or its portion in the fusion peptide) is a short peptide with 5 to 100 amino acids in length, preferably 8 to 80 or 12 to 60 amino acids in length.

The entire fusion peptide in any embodiment can be a peptide with 15 to 500 amino acids in length, preferably 15 to 200 amino acids in length, even more preferred 18 to 100 amino acids in length.

The Cbl-b binding peptides that bind to the TKB domain of Cbl-b, thereby inhibiting Cbl-b activity can be administered to a cell in any form, conjugate or composition. These forms, conjugates or compositions for administration allow entry of the Cbl-b peptide into a living cell, thereby passing a cell membrane. Preferred are conjugates with cell delivery or cell penetrating moieties, such as fusion peptides with cell penetrating peptides. Conjugates and fusion peptides may be connected by a linker moiety or linker peptide. Such linkers are usually small, e.g. less than 10kDa, or less than 5 kDa, or in the range of 0, or 1 to 20 amino acids in length. Other forms for cellular administration include vesicles, micelles, liposomes or microsomes.

Interestingly, a fusion peptide binding to the TKB domain of Cbl-b (Fig. 11e) significantly improved immune function in response to *Candida albicans* stimulation (Fig. 11g, h; Fig. 12b). The present invention, in a further aspect, therefore relates to a fusion peptide comprising a cell delivery portion and a Cbl-b binding peptide (in particular as defined above), wherein the Cbl-b binding peptide binds to the TKB domain of Cbl-b. In particular, the fusion peptide comprises or consists of a cell delivery portion and a Cbl-b binding peptide, wherein the Cbl-b binding peptide binds to the TKB domain of Cbl-b. The cell delivery portion may be a cell penetrating peptide. Possible routes of cellular delivery are fusion of vesicles etc. with a cell membrane, penetration through the lipid bilayer, translocation or endocytosis.

Cell penetrating peptides (CPPs) are short peptides, usually cationic or amphipathic, that mediate the intracellular delivery of a variety of biological cargos (Bechara et al., 2013; Jones et al., 2005, en.wikipedia.org/wiki/Cell-penetrating_peptide, all incorporated herein by reference). Peptides derived from proteins, chimeric peptides and synthetic CPPs are the three main classes of CPPs that can be distinguished (Bechara et al., 2013). Having important advantages such as low toxicity, CPPs have become common biotechnological tools for cross-cellular transport of biologically active peptides *in vitro* and *in vivo* (Jones et al., 2005; Lindsay et al., 2002). Thus, in a preferred embodiment, the cell delivery portion of the fusion peptide is a cell penetrating peptide. Important representatives of CPPs are Antennapedia, Tat, Transportan, dNP1, dNP2 (Lim et al., 2015) or Polyarginine. Antennapedia has been shown to be especially useful for *in vitro* peptide delivery, taking into account both the magnitude of uptake and cytotoxicity (Jones et al., 2005). Especially effective are CPPs comprising two, three, four, five, six, seven or more cations, especially cationic amino acid residues, such as H, K or R, preferably selected from K or R. Preferably, the CPP is selected from DRQIKIWFQNRRMKWKK (SEQ ID NO: 10), GRKKRRQRRRPPQ (SEQ ID NO: 11), GWTLNSAGYLLGKINLKALAALAKKIL (SEQ ID NO: 12), (KKDKKDERRX_{A}K)ₘ (SEQ ID NO: 13), wherein 1≤m≤5 and X_{A} is selected from K or R, and (KIKKX_{B}KKKGRK)ₘ (SEQ ID NO: 14), wherein 1≤m≤5 and X_{B} is selected from V, I or T, or (R)ₙ, wherein 6≤n≤12. Preferably the CPP (or its portion in the fusion peptide) is a short peptide with 8 to 100 amino acids in length, preferably 10 to 80 or 12 to 60 amino acids in length.

In another preferred embodiment, the Cbl-b binding peptide of the fusion peptide is of or derived from Syk, as described above. Preferably, the Cbl-b binding peptide is of Syk and the peptide is a regulator of innate immunity. The peptide is preferably a regulator of innate anti-parasitic immunity or anti-fungal immunity.

Other proteins or peptides binding to the TKB domain of Cbl-b can be used, such as proteins or Cbl-b binding peptides of the insulin receptor substrate 1 (IRS-1), a key intermediate of skeletal muscle growth regulated by insulin-like growth factor (IGF-1) (Nakao et al., 2009; Ohno et al., 2016) or the Zeta-associated protein of 70 kDa (Zap-70), playing a role in T-cell development and lymphocyte activation. In addition, residues located in receptor tyrosine kinases (RTKs) such as EGFR, Met and CSF1 receptors that interact with the TKB domain of Cbl-b can be used (Mancini et al., 2002; Nakao et al., 2009; Ohno et al., 2016; Peschard et al., 2001; Peschard et al., 2004; Tsygankov et al., 2001). Some of the TKB binding sites of Cbl-b share a sequence motif comprising the phosphotyrosine. Thus, the Cbl-b binding peptide preferably has the sequence motif N/DXpY, wherein X is selected from any amino acid, preferably containing proteinogenic and/or non-proteinogenic amino acids as described above. N/D is either asparagine (N) or aspartic acid (D), and pY is phosphotyrosine. More preferably, the Cbl-b binding peptide has the sequence motif N/DX₁pYX₂P (SEQ ID NO: 15), wherein X₁ and X₂ are selected from any amino acid, preferably containing proteinogenic and/or non-proteinogenic amino acids as described above. N/D is either asparagine (N) or aspartic acid (D), and pY is phosphotyrosine. Even more preferred, the Cbl-b binding peptide has a sequence length of 5 to 20 amino acids and/or a sequence identity to Syk of at least 80%, preferably at least 90%. In a further embodiment, the Cbl-b binding peptide is a fragment of Zap-70, or IRS-1. In Zap-70 of mice und humans, the homologous sequence of the sequence motif N/DX₁pYX₂P (SEQ ID NO: 15) is DgpYTP (SEQ ID NO: 16) in position 288-292 in mice (mouse Zap-70²⁸⁸⁻²⁹²) and in position 290-294 in humans (human Zap-70 ²⁹⁰⁻²⁹⁴). In IRS-1 of mice and humans, the homologous sequence of the sequence motif N/DX₁pYX₂P is DgpYMP (SEQ ID NO: 17) in position 606-610 in mice (mouse IRS-1⁶⁰⁶⁻⁶¹⁰) and in position 610-614 in humans (human IRS-1⁶¹⁰⁻⁶¹⁴). Thus, in a further preferred embodiment, the Cbl-b binding peptide comprises or consists of DGpYTP or DgpYMP.

Preferably, the Cbl-b binding peptide comprises a tyrosine, wherein the tyrosine is phosphorylated. Phosphorylating the tyrosine increases interaction with Cbl-b and is a preferred embodiment in all aspects of the invention.

The invention further relates to a pharmaceutical composition, comprising the fusion peptide or the Cbl-b binding peptide, and a pharmaceutically acceptable carrier. The carrier may promote cellular uptake of said peptide.

In one preferred embodiment, the pharmaceutically acceptable carrier is selected from vesicles, micelles, liposomes and microsomes. These may increase cellular uptake of the peptide, for therapeutic or also non-therapeutic uses. Pharmaceutical compositions can comprise pharmaceutically acceptable salts, buffers, tonicity agents or pharmaceutically acceptable carriers. Pharmaceutical carriers provide improved compatibility of the composition and enable improved solubility as well as improved bioavailability of the active ingredients. Examples are emulsifiers, thickening agents, redox components, starch, alcohol solutions, polyethylene glycol or lipids. In another preferred embodiment, the pharmaceutical composition is prepared or used for parenteral administration, especially intravenous, intraarterial, intramuscular, intrathecal, subcutaneous or intraperitoneal administration. In another preferred embodiment, the pharmaceutical composition is prepared or used for topical administration, preferably further comprises a skin penetration enhancer. Suitable skin penetration enhancers are selected from the group comprising C12-C15 alkyl benzoate, pentylene glycol, polyethylene glycol, ethoxydiglycol, dimethyl sulfoxide, sodium lauryl sulfate, polysorbate polyethylene sorbitan monolaurate, lecithin, and mixtures thereof.

Preferably, the peptide or pharmaceutical composition is for use in therapy, wherein the peptide or composition is administered to a patient. More preferably, the peptide or pharmaceutical composition is for use in the prevention or treatment of a fungal infection in the patient.

Toll-like receptors (TLRs) and CLRs have been shown to recognize conserved structures of invading pathogens, termed pathogen-associated molecular patterns (PAMPs), and thus induce immune responses (Kawai and Akira 2010; Kawai and Akira, 2011). Chiba et al. (2014) demonstrated that the CLR Dectin-1 recognizes tumor cells and, as a consequence, activates innate immune cells for anti-tumor responses. Dectin-1 serves as receptor for glyco-epitopes specifically expressed by tumor cells. Due to the function of Cbl-b in negative regulation of proximal Dectin-1 signaling, the TKB-binding peptide enhances Dectin-1 signal transduction impacting on the immune recognition of tumor cells by the innate immune system. In addition to the increase in activation of the immune system by the inventive inhibition of Cbl-b, this mechanism further enhances anti-tumor immunity driven by the innate immune system. Therefore, in a further preferred embodiment of the invention, the peptide or pharmaceutical composition is for use in the prevention or treatment of tumors, cancer, viral infections, bacterial infections, parasitic infections, especially intracellular parasites, mycosis, or drug-induced immunosuppression in a patient. Further disease or conditions to be treated are characterized by a congenital or acquired immune insufficiency, such as AIDS, multiple myeloma, chronic lymphatic leukemia, drug-induced immunosuppression or a cancer, optionally with the selection of disease-specific antigens. Treatment of a cancer involving solid tumors is preferred in particular.

N-glycan structures are highly expressed in tumor cells. In another preferred embodiment of the invention, the peptide or pharmaceutical composition is for use in the prevention or treatment of such tumors, wherein the tumor cells express N-glycan at high amounts. High levels of N-glycan amounts (molar) in a particular cell type are at least 1.5-fold of the N-glycans found in said non-tumor cell type. These non-tumor cell types are e.g. melanocytes, epithelial cells, lymphatic cells, mesenchymal cells, lung cells, blood cells, liver cells. According to preferred embodiments of the invention, the cancer or tumor disease to be treated according to the present invention is selected from ovarian cancer, testicular cancer, prostate cancer, breast cancer; cancer diseases of the digestive tract, in particular stomach cancer, colon cancer, rectal cancer, pancreatic cancer, esophageal cancer and liver cancer; kidney cancer, skin cancer, in particular melanoma, basal cell carcinoma and squamous cell carcinoma; neuroblastoma and glioblastoma, lung cancer, thyroid cancer, sarcoma, head and neck cancer, squamous cell carcinoma, lymphoma and leukemia (wherein the terms "cancer", "tumor", "carcinoma" etc. are always used interchangeably herein and refer to malignant diseases).

Preferably, the peptide or pharmaceutical composition for use is administered to the patient by intraperitoneal injection, comprising 1 mg to 500 mg of the fusion peptide or Cbl-b binding peptide, preferably for up to 7 days, 14 days, 21 days or 28 days and/or daily. Preferred doses are also 5 mg to 200 mg and 10 mg to 100 mg.

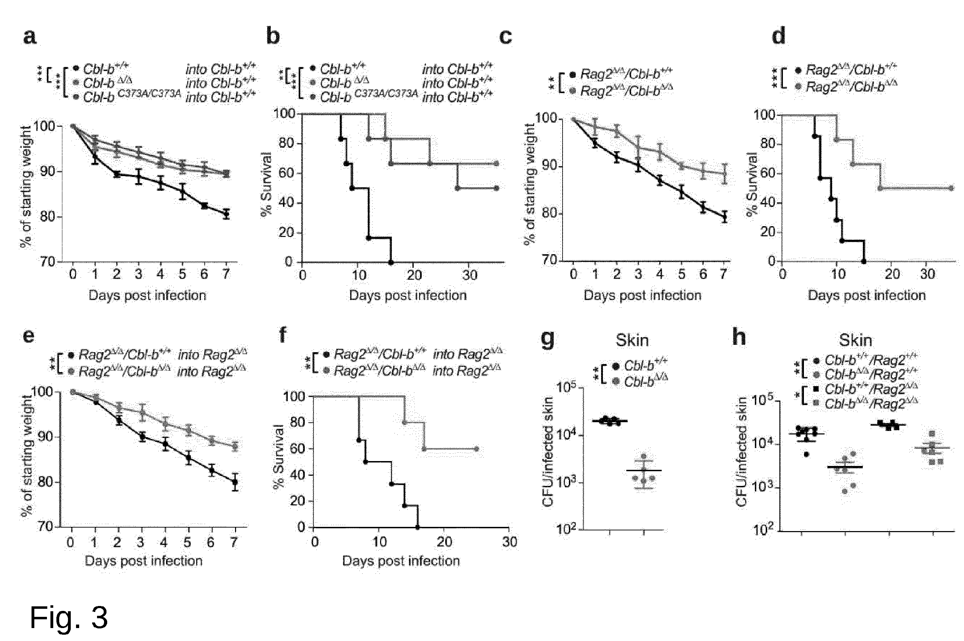

Cbl-b is a negative regulator of the innate and adaptive immune system (Fig. 3). Thus, the Cbl-b binding peptide as defined herein can positively stimulate both innate and adaptive immune reactions when administered as a vaccine adjuvant. Specifically, the Cbl-b binding peptide as defined herein can lift checkpoint blockades during vaccination. Therefore, the present invention also provides a Cbl-b inhibitor for use as a vaccine adjuvant in a patient, wherein the Cbl-b inhibitor is a Cbl-b binding peptide as defined herein. In one embodiment, the vaccination is against tumors, or against viral, bacterial or parasitic infections. In an associated embodiment, the invention provides a vaccine, comprising as an adjuvant a Cbl-b inhibitor. Preferably, the Cbl-b inhibitor is a Cbl-b binding peptide of Syk, and at least one antigen.

"Vaccination" as used herein is not to be understood in the absolute sense - i.e., administration of an immunogen which leads to absolute protection by the immune system - but rather as immunological administration to increase protection by the immune system and/or to activate the immune system, in particular the cells thereof against the vaccine antigen. The inventive Cbl-b inhibitor, especially the inventive peptide, increase the immunoreactivity to a vaccine antigen in a patient and/or increasing the immunoreactivity per se in patient.

In a preferred embodiment, the patient to be treated is a mammal. In an even more preferred embodiment, the patient is a human. In another embodiment, the Cbl-b inhibitor for use as a vaccine adjuvant is administered to the patient together with the vaccine that comprises the antigen against which an immune reaction shall be increased by Cbl-b inhibition. In an alternative embodiment, the Cbl-b inhibitor is administered to the patient before and/or after administration of the vaccine.

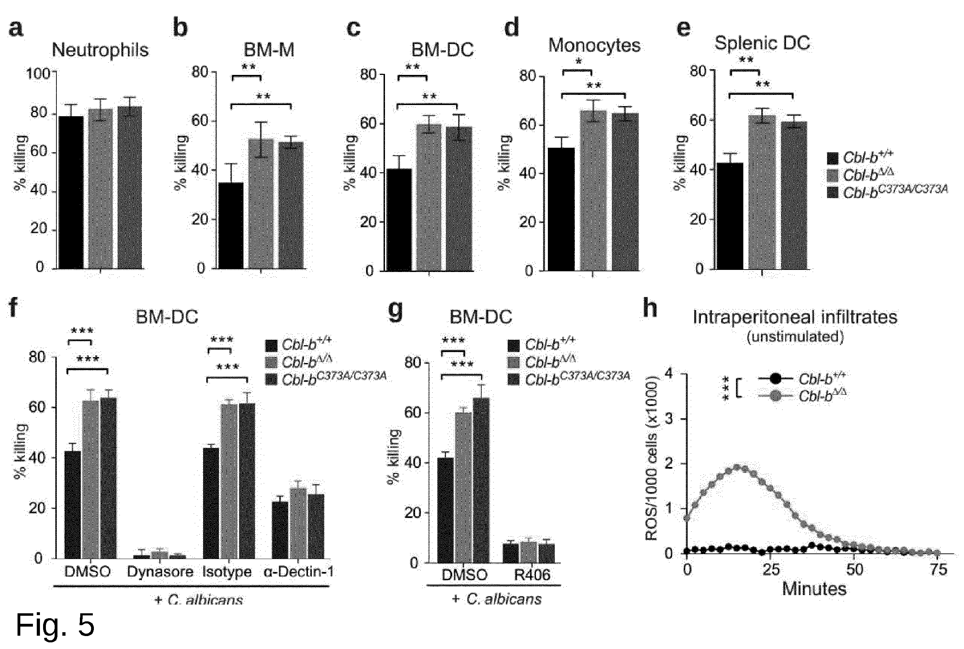

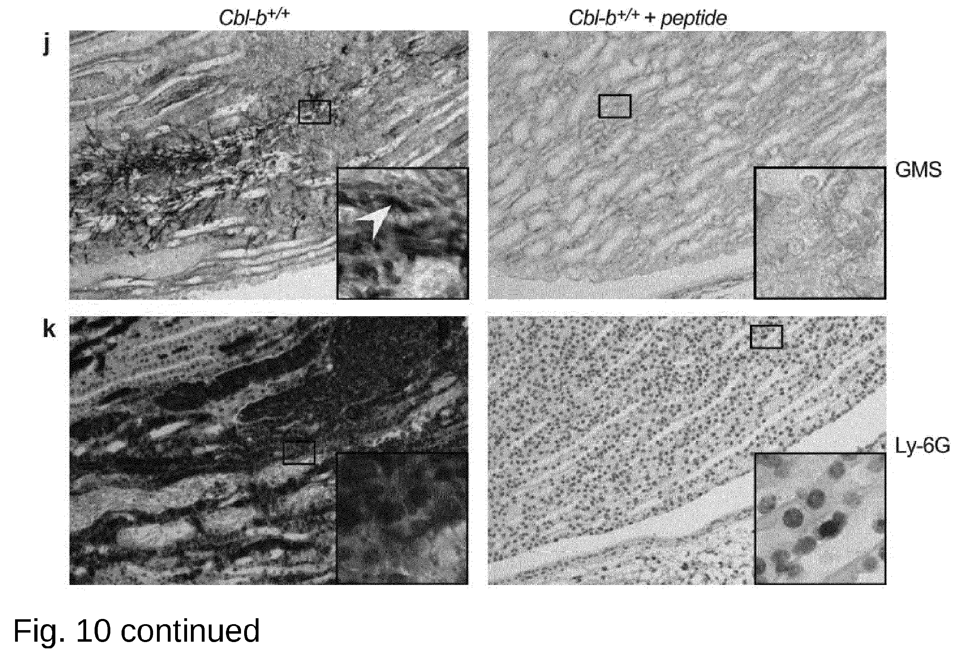

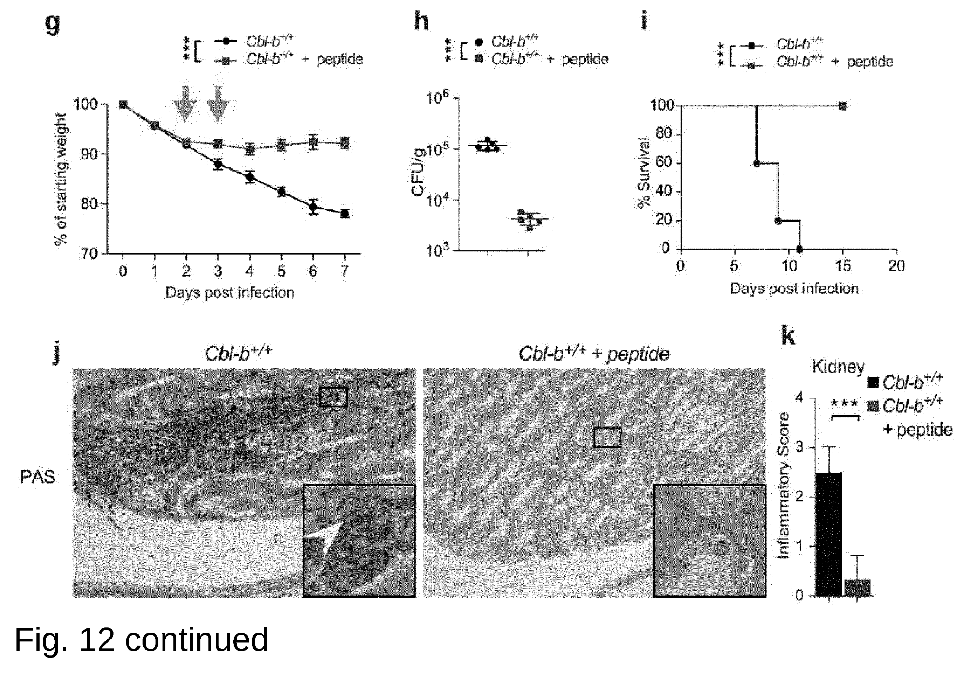

It has been shown by the present invention that inhibition of Cbl-b is effective in the treatment of both systemic and cutaneous fungal infections, preferably candidiasis (e.g. example 3, Fig. 5h). A cutaneous fungal infection can further be a dermaphytosis, and a systemic fungal infection can also be an aspergillosis. Cutaneous fungal infections can be treated by topical therapy. Topical anti-fungal administration forms include creams, ointments, gels, lotions, powders, solutions or sprays. It has been further demonstrated in the present invention that treatment with the fusion peptide as described herein is effective in the treatment of already established fungal sepsis (Example 6, Fig. 10j, k and 12g-k). Fungal sepsis has been reported to be increasing and to be associated with considerable morbidity and mortality (Lepak and Andes, 2011). In a preferred embodiment, the fungal infection to be treated with the inventive Cbl-b inhibitor, peptide or composition is a dermatophyte or an aspergillus infection. Preferably, the fungal infection is acute, systemic or cutaneous.

Preferably, the patient has or is at risk of developing sepsis, especially sepsis due to a fungal infection ("fungal sepsis"). The inventive increase in immune stimulation, especially of immune reactions against pathogens, may prevent or reduce the risk of sepsis. In an acute sepsis, it can quicken immune clearance.

The disease or condition to be treated may be acute or chronic. Chronic conditions are defined to last at least 90 days. Acute treatment may commence on days 0 to 89 after onset of the disease or condition, preferably on days 4 to 30.

In preferred embodiments of the present invention, the reduction or inhibition of the function of Cbl-b is transient, i. e. the function is only temporarily reduced and can therefore recover again, e. g. by consumption or degradation of inhibitors. The transient reduction of Cbl-b in immune cells *in vitro* or in a patient can also be performed in a repetitive manner, e. g. until a therapeutic success has been achieved.

In a further preferred embodiment of the invention, the Cbl-b inhibitor or peptide or pharmaceutical composition is administered to the patient intravenously, intraarterially, intramuscularly, intravascularly, subcutaneously, or intraperitoneally. More preferably, the patient to be treated is a mammal. Even more preferably, the patient is a human.

In a further preference, the Cbl-b inhibitor or peptide for use is administered to the patient using a pharmaceutically acceptable carrier, especially vesicles, micelles, liposomes or microsomes.

The present invention therefore also relates to a method for regulating, especially increasing, innate immunity, comprising the inhibition of Cbl-b at least in immune cells, e.g. in a patient as described above. Preferably, a fusion peptide or Cbl-b binding peptide as disclosed herein are administered to the patient. More preferably, the innate immunity is innate anti-parasitic immunity, innate anti-fungal, or innate anti-tumor immunity.

Immune cells targeted or affected by the inventive treatment are preferably antigen-presenting cells, PBMCs (peripheral blood mononuclear cells), T-lymphocytes, B-lymphocytes, monocytes, macrophages and/or dendritic cells, in particular T-lymphocytes, CD8+ T-lymphocytes, CD4+ T-lymphocytes, in particular Th1, Th2, Th17, Tregs (regulatory T-cells) or CTL (cytotoxic T-cells) or NK cells.

The present invention further relates to a kit comprising the peptide and a pharmaceutically acceptable excipient. Preferably, the kit is suitable for intracellular administration in the patient. Any route of administration to the patient as described above may be selected. The kit may comprise a container comprising the compounds, e.g. peptides or Cbl-b inhibitor of the invention and auxiliary substances of a pharmaceutical composition or other reagents to treat cells, the kit may be used in any method or any uses of the invention. The container and/or the kit may also comprise a further immunostimulating substance (apart from the peptide of the invention), preferably cytokine(s) or ligands of other receptors (e.g., TLRs) or antibodies to surface molecules, preferably CD3 and/or CD28, to additionally enhance the stimulation. Likewise, the kit or the container may also comprise stabilizing components, media or buffers.

In the kit or in a combinatory treatment of the patient or of cells, *in vitro* or *in vivo,* the Cbl-b inhibitor of the invention may be combined with further immune stimulating compounds such as an immune cell-stimulatory cytokine, e. g. a cytokine selected from the common gamma-chain cytokines, in particular IL-2, IL-15 and IL-21; cytokines that stimulate both the cells of the adaptive and of the innate immune system, in particular IL-12, IL-23 and IL-27; effector cell cytokines, such as IL-1, IL-17 and IL-18; an interferon, in particular interferon-alpha; or an interferon stimulator; an antibody, in particular an antibody which recognizes tumor cell surface molecules or a TLR or PAMP receptor ligand, in particular agonists, preferably of TLR-1, TLR-2, TLR-3, TLR-7, TLR-8 and TLR-9. An immune cell-stimulatory cytokine, a cytokine of both the adaptive and the innate immune system, an effector cell cytokine or an interferon stimulator according to the present invention are preferably selected from the group comprising IL-1, IL-2, IL-3, IL-4, IL-5, IL-6, IL-7, IL-8, IL-9, IL-11, IL-12, IL-13, IL-14, IL-15, IL-16, IL- 17a, IL-17f, IL-18, IL-19, IL-20, IL-21, IL-22, IL-23, IL-24, IL-25, IL-26, IL-27, IL-28, IL-29, IL-30, IL-31, IL-32, IL-33, IL-34, IL-35, IFN-alpha, IFN-beta, IFN-gamma, IFN-lambda, TNF (formerly TNF-alpha) and Lymphotoxin-alpha (formerly TNF-beta). The terms "simultaneous" or "together with" or "in combination with" or "combined with" as used in the context of the administration of the substances according to the present invention refer to the administration of at least one immune stimulating compound other than the Cbl-b inhibitor. If the administration is carried out using a plurality of different pharmaceutical compositions, the coadministration may be conducted simultaneously or sequentially.

Cbl-b acts in innate immune cells, especially in macrophages and dendritic cells, *in vitro* and that inhibition of Cbl-b increased immune response or activity of immune cells. Thus the invention relates to activating immune cells. The immune cells may be antigen-presenting cells, PBMCs (peripheral blood mononuclear cells), T-lymphocytes, B-lymphocytes, monocytes, macrophages and/or dendritic cells, in particular T-lymphocytes, CD8+ T-lymphocytes, CD4+ T-lymphocytes, in particular Th1, Th2, Th17, Tregs (regulatory T-cells) or CTL (cytotoxic T-cells) or NK cells. Such cells can also be treated and stimulated with the inventive Cbl-b inhibitor. The present invention therefore provides an *in vitro* method for stimulating or activating an immune cell, comprising contacting or treating the cell with a fusion peptide or a Cbl-b binding peptide as disclosed herein. Preferably, the method comprises the production of cytokines by the activated or stimulated immune cells. E.g., the produced cytokines are selected from IL-6, IL-1β, IL-23 and TNF.

The term "treating" or "treatment" as used herein means to cure an already present disease state or condition or to increase the likelihood of recovery from the disease state or condition. Treating can also include inhibiting, i.e. arresting the development of a disease state or condition, and ameliorating, i.e. causing regression of a disease. Treatment can be to ameliorate disease symptoms without curing a patient.

The term "preventing" or "prevention" as used herein does not mean to stop a disease state or condition from occurring in a patient or subject completely but may also refer to a reduced risk of developing a disease state or condition.

In a preference, the pharmaceutical composition of the present invention can further comprise one or more excipients pharmaceutically acceptable for parenteral administration. Suitable excipients are known to the person skilled in the art, for example water (especially water for injection), saline, Ringer's solution, dextrose solution, buffers, Hank solution, vesicle forming compounds (e.g. lipids), fixed oils, ethyl oleate, 5% dextrose in saline, substances that enhance isotonicity and chemical stability, buffers and preservatives. This pharmaceutical composition can (as a drug) be administered via appropriate procedures known to the skilled person to a patient in need thereof (i.e. a patient having or having the risk of developing the diseases or conditions mentioned herein). The preferred route of administration of said pharmaceutical composition is parenteral administration, especially through intravenous, intraarterial, intramuscular, intrathecal, subcutaneous or intraperitoneal administration. For parenteral administration, the pharmaceutical composition of the present invention is provided in injectable dosage unit form, e.g. as a solution, suspension or emulsion, formulated in conjunction with the above-defined pharmaceutically acceptable excipients. The dosage and method of administration, however, depends on the individual patient to be treated. Said pharmaceutical composition, can be administered in any suitable dosage known from other peptide dosage regimens or specifically evaluated and optimized for a given individual. For example, the active agent (i.e. the fusion peptide of the present invention or the Cbl-b binding peptide) may be present in the pharmaceutical composition in an amount from 1 mg to 10 g, preferably 50 mg to 2 g, in particular 100 mg to 1 g. Usual dosages can also be determined on the basis of kg body weight of the patient, for example preferred dosages are in the range of 0.1 mg to 100 mg/kg body weight, especially 1 to 10 mg/kg body weight (per administration session). The administration may occur e.g. once daily, once every other day, once per week or once every two weeks. As the preferred mode of administration of said pharmaceutical composition, is parenteral administration, the pharmaceutical composition according to the present invention is preferably liquid or ready to be dissolved in liquid such sterile, de-ionised or distilled water or sterile isotonic phosphate-buffered saline (PBS). Preferably, 1000 µg (dry-weight) of such a composition comprises or consists of 0.1-990 µg, preferably 1-900µg, more preferably 10-200µg active agent according to the present invention, and optionally 1-500 µg, preferably 1-100 µg, more preferably 5-15 µg (buffer) salts (preferably to yield an isotonic buffer in the final volume), and optionally 0.1-999.9 µg, preferably 100-999.9 µg, more preferably 200-999 µg other excipients. Preferably, 100 mg of such a dry composition is dissolved in sterile, de-ionised/distilled water or sterile isotonic phosphate-buffered saline (PBS) to yield a final volume of 0.1-100 ml, preferably 0.5-20 ml, more preferably 1-10 ml.

Herein, the term "binding" or "binds" entails that molecule A (such as a molecule binding to Cbl-b, such as the Cbl-b binding peptide) has a dissociation constant (also called "affinity") in regard to molecule B (such as Cbl-b, specifically the TKB domain thereof) that is lower than (i.e. "stronger than") 1000 nM, preferably lower than 100 nM, more preferably lower than 50 nM, even more preferably lower than 10 nM, especially lower than 5 nM.

Preferably, the patient to be prophylactically or therapeutically treated is a mammal, especially a human. Typically, the patient (who preferably has a fungal infection as defined herein, and especially has been diagnosed with a fungal infection as defined herein) is in need of the inventive treatment. In the context of the inventive treatment for prevention of a fungal infection, the patient may be a patient that does not have the fungal infection but is at risk of developing the fungal infection, such as an immune-compromised patient.

Herein, the term "TKB binding peptide" refers to a Cbl-b binding peptide which binds to the TKB domain of Cbl-b.

"Comprising" is used as an open ended term and means that other elements may also be present. "Consisting of" is used as a closed term, wherein, accordingly the described element or group of elements of the invention consist only of the recited element(s). An embodiment of the invention that has been described as comprising a certain element or group or elements, if not stated otherwise, may also only consist of this element or group of elements.

The use of the word "a" or "an" when used in conjunction with the term "comprising" in the claims and/or the specification may mean "one", but it is also consistent with the meaning of "one or more", "at least one", and "one or more than one".

Following embodiments further define the present invention:
1. A fusion peptide comprising a cell delivery portion and a casitas b-lineage lymphoma-b (Cbl-b) binding peptide, wherein the Cbl-b binding peptide binds to the tyrosine kinase binding (TKB) domain of Cbl-b.
2. The fusion peptide according to 1, wherein the cell delivery portion is a cell penetrating peptide.
3. The fusion peptide according to 1 or 2, wherein the Cbl-b binding peptide is a peptide fragment of spleen tyrosine kinase (Syk).
4. The fusion peptide according to any one of 1 to 3, wherein the Cbl-b binding peptide has the sequence motif N/DXpY, wherein X is selected from any amino acid, N/D is either asparagine (N) or aspartic acid (D), and pY is phosphotyrosine.
5. The fusion peptide according to any one of 1 to 4, wherein the Cbl-b binding peptide has the sequence motif N/DX₁pYX₂P, wherein X₁ and X₂ are selected from any amino acid, preferably containing proteinogenic and/or non-proteinogenic amino acids as described above, N/D is either asparagine (N) or aspartic acid (D), and pY is phosphotyrosine.
6. The fusion peptide according to any one of 1, 2, 4 and 5, wherein the Cbl-b binding peptide is a peptide fragment of zeta-associated protein of 70 kDa (Zap-70) or insulin receptor substrate 1 (IRS-1).
7. The fusion peptide according to any one of 1 to 6, wherein the Cbl-b binding peptide binds to Cbl-b, the peptide comprising a tyrosine, wherein the tyrosine is phosphorylated.
8. The fusion peptide according to any one of 1 to 7, comprises the following consensus sequence:

   SFNPYEPX₁X₂X₃,

   wherein X₁-X₃ are selected independently from any amino acid, preferably containing proteinogenic and/or non-proteinogenic amino acids as described above, more preferably wherein the Cbl-b binding peptide is a peptide fragment of Syk.
9. The fusion peptide according to 8, wherein the Cbl-b binding peptide comprises the sequence SFNPYEPTGG.
10. The fusion peptide according to 8, wherein wherein the Cbl-b binding peptide comprises the sequence SFNPYEPTGG.
11. The fusion peptide according to 8, wherein the Cbl-b binding peptide comprises the sequence SFNPYEPEVA.
12. The fusion peptide according to 8, wherein the Cbl-b binding peptide comprises the sequence SFNPYEPELA.
13. The fusion peptide according to any one of 1 to 12, wherein the Cbl-b binding peptide is from Syk and comprises, preferably consists of, the following consensus sequence:

   STVSFNPYEPX₁X₂X₃X₄WX₅,

   wherein X₁-X₅ are selected independently from any amino acid, preferably containing proteinogenic and/or non-proteinogenic amino acids as described above, more preferably wherein the Cbl-b binding peptide is a peptide fragment of Syk.
14. The fusion peptide according to 13, wherein the Cbl-b binding peptide comprises, preferably consists of, the sequence STVSFNPYEPTGGPWG.
15. The fusion peptide according to 13, wherein the Cbl-b binding peptide comprises, preferably consists of, the sequence STVSFNPYEPTGGAWG.
16. The fusion peptide according to 13, wherein the Cbl-b binding peptide comprises, preferably consists of, the sequence STVSFNPYEPEVAPWA.
17. The fusion peptide according to 13, wherein the Cbl-b binding peptide comprises, preferably consists of, the sequence STVSFNPYEPELAPWA.
18. The fusion peptide according to any one of 8 to 17, wherein the tyrosine in said sequence is phosphorylated.
19. The fusion peptide according to any one of 2 to 18, wherein the cell penetrating peptide is selected from DRQIKIWFQNRRMKWKK, GRKKRRQRRRPPQ, GWTLNSAGYLLGKINLKALAALAKKIL, (KKDKKDERRX_{A}K)ₘ, wherein 1≤m≤5 and X_{A} is selected from K or R, and (KIKKX_{B}KKKGRK)ₘ, wherein 1≤m≤5 and X_{B} is selected from V, I or T, or (R)ₙ, wherein 6≤n≤12.
20. A fusion peptide comprising a cell delivery portion as defined in any one of 1 to 19 and a Cbl-b binding peptide, wherein the Cbl-b binding peptide is an intramer or aptamer, wherein the Cbl-b binding peptide inhibits Cbl-b, preferably wherein the Cbl-b binding peptide binds to the TKB domain of Cbl-b.
21. The peptide according to any one of 1 to 20, wherein the Cbl-b binding peptide is of Syk and wherein the peptide is a regulator of innate immunity.
22. The peptide according to any one of 1 to 21, wherein the Cbl-b binding peptide is of Syk and wherein the peptide is a regulator of innate anti-parasitic, anti-fungal or anti-tumor immunity.
23. A pharmaceutical composition, comprising the fusion peptide or the Cbl-b binding peptide as set forth in any one of 1 to 22, and a pharmaceutically acceptable carrier promoting cellular uptake of said peptide.
24. The pharmaceutical composition according to 23, wherein the pharmaceutically acceptable carrier is selected from vesicles, micelles, liposomes and microsomes.
25. The pharmaceutical composition according to 23 or 24, prepared for parenteral administration, especially intravenous, intraarterial, intramuscular, intrathecal, subcutaneous or intraperitoneal administration.
26. The pharmaceutical composition according to 23 or 24, prepared for topical administration, preferably further comprising a skin penetration enhancer.
27. The peptide or pharmaceutical composition according to any one of 1 to 26, for use in therapy, wherein the peptide or composition is administered to a patient.
28. The peptide or pharmaceutical composition for use according to 27 for use in the prevention or treatment of a fungal infection in the patient.
29. The peptide or pharmaceutical composition for use according to 27 or 28, wherein the peptide is a Cbl-b binding peptide of Syk.
30. The peptide or pharmaceutical composition for use according to any one of 27 to 29 for use in the prevention or treatment of tumors, cancer, viral infections, bacterial infections, parasitic infections, especially intracellular parasites, mycosis, or drug-induced immunosuppression in a patient.
31. The peptide or pharmaceutical composition for use according to any one of 27 to 30, wherein the peptide is administered to the patient in a dose of 1 mg to 500 mg of the fusion peptide or Cbl-b binding peptide, preferably for up to 7 days, 14 days, 21 days or 28 days and/or daily.
32. A Cbl-b inhibitor for use in therapy in a patient as a vaccine adjuvant, preferably wherein the Cbl-b inhibitor is a Cbl-b binding peptide, more preferably a Cbl-b binding peptide of Syk.
33. A Cbl-b inhibitor for use in the prevention or treatment of a fungal infection in a patient, wherein the fungal infection is a yeast infection, preferably a candidiasis or a cryptococcosis.
34. A Cbl-b inhibitor for use in the prevention or treatment of a fungal infection in a patient, wherein the Cbl-b inhibitor comprises a Cbl-b binding peptide of Syk.
35. The Cbl-b inhibitor or peptide or pharmaceutical composition for use according to any one of 27 to 34, wherein the fungal infection is a yeast infection, preferably a candidiasis, especially a *Candida albicans, Candida glabrata, Candida krusei,* or a *Candida dubliniensis* infection.
36. The Cbl-b inhibitor or peptide or pharmaceutical composition for use according to 27 to 34, wherein the fungal infection is a dermatophyte or an aspergillus infection.
37. The Cbl-b inhibitor or peptide or pharmaceutical composition for use according to any of 27 to 36, wherein the fungal infection is acute, systemic or cutaneous.
38. The Cbl-b inhibitor or peptide or pharmaceutical composition for use according to any one of 27 to 37, wherein the patient has or is at risk of developing fungal sepsis.
39. The Cbl-b inhibitor or peptide or pharmaceutical composition for use according to any one of 27 to 38, wherein the Cbl-b inhibitor, peptide or composition is administered to the patient intravenously, intraarterially, intramuscularly, intravascularly, subcutaneously, intraperitoneally, intraperitoneally or intrathecally.
40. The Cbl-b inhibitor, peptide or pharmaceutical composition for use according to any one of 27 to 39, wherein the patient is a mammal, preferably a human.
41. The Cbl-b inhibitor or peptide for use according to any one of 27 to 40, wherein the inhibitor or peptide is administered to the patient using a pharmaceutically acceptable carrier, especially vesicles, micelles, liposomes or microsomes.
42. A vaccine, comprising as an adjuvant a Cbl-b inhibitor, preferably wherein the Cbl-b inhibitor comprises a Cbl-b binding peptide of Syk, and at least one antigen, preferably the Cbl-b inhibitor being defined as in any one of 1 to 22 and 27 to 40.
43. A method for regulating, especially increasing, innate immunity, comprising the inhibition of Cbl-b, preferably by administering to a patient the fusion peptide or Cbl-b inhibitor as set forth in any one of 1 to 22 and 27 to 40.
44. The method according to 43, wherein the innate immunity is anti-parasitic, anti-fungal immunity, or anti-tumor immunity.
45. A kit comprising the peptide according to 1 to 31 and a pharmaceutically acceptable excipient, preferably suitable for intracellular administration in the patient.
46. An *in vitro* or *in vivo* method for stimulating or activating an immune cell, comprising contacting or treating the cell with a fusion peptide or a Cbl-b binding peptide as set forth in any one of 1 to 22.
47. The *in vitro* or *in vivo* method for stimulating or activating an immune cell according to 46, wherein cytokines are produced, wherein the cytokines are preferably selected from IL-6, IL-1β, IL-23 and TNF.

The present invention is further illustrated by the following figures and examples, without being restricted thereto.

### Figures

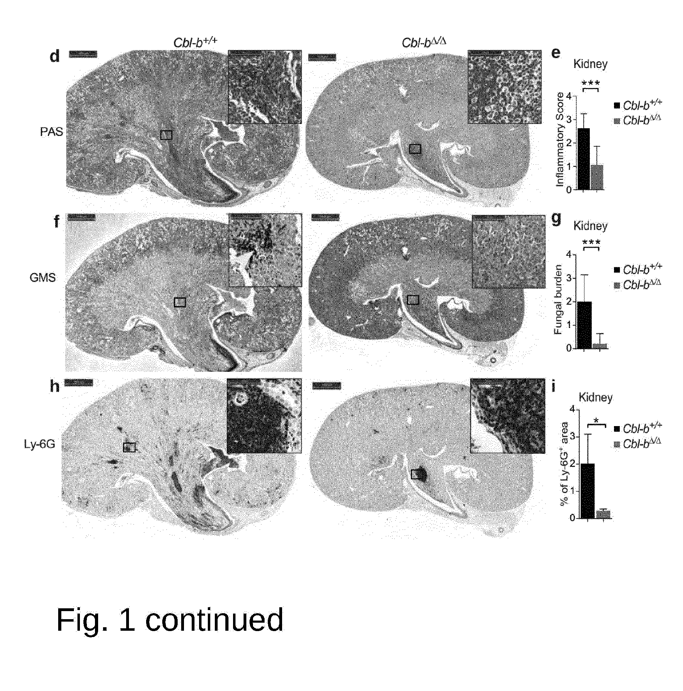

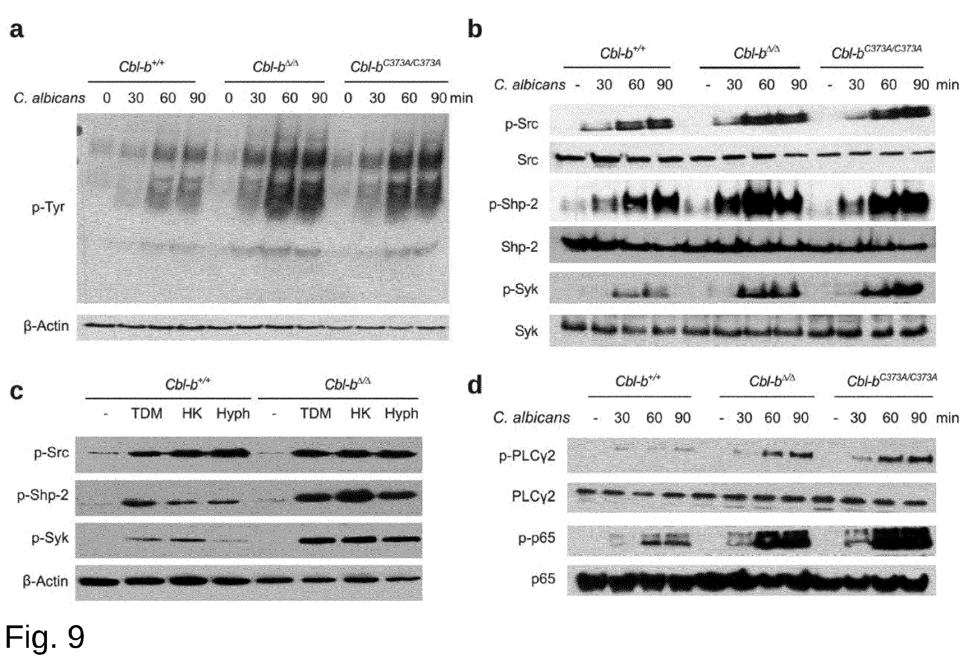

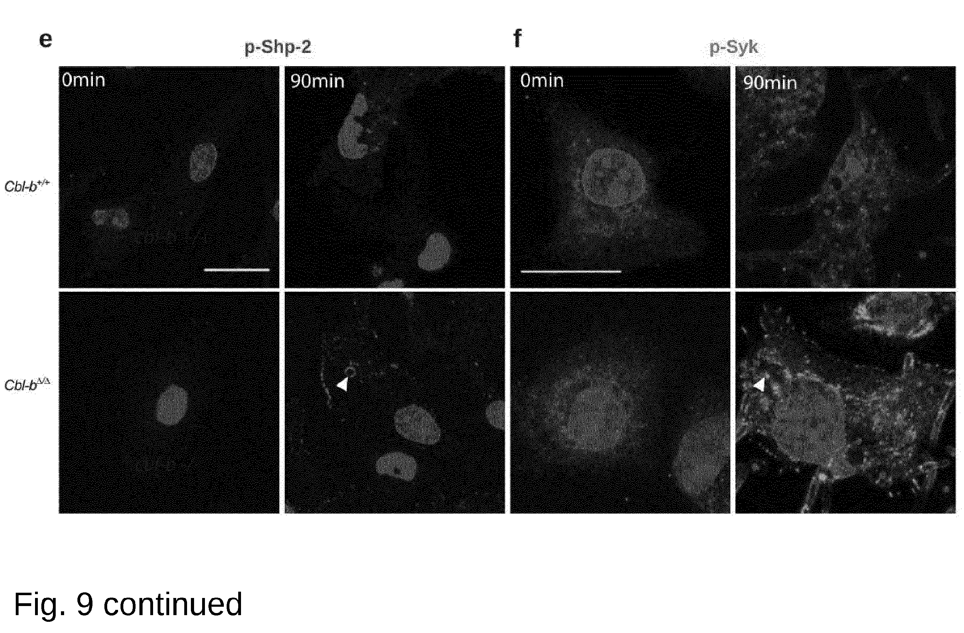

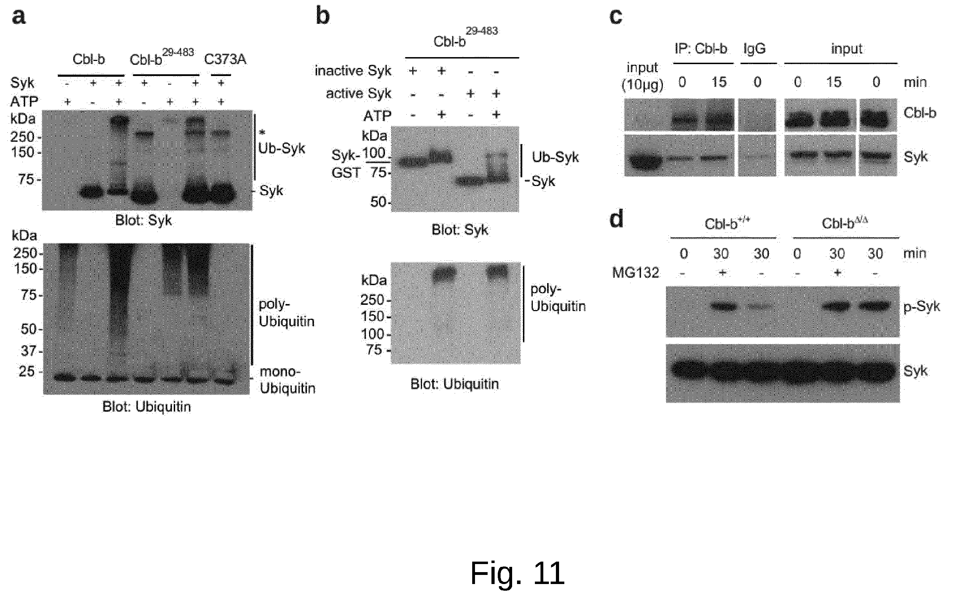

**Fig. 1****: Cb1-b regulates anti-fungal immunity.**
   **(a,b)** *Cbl-b*^{*+*/}*⁺, Cbl-b*^{*Δ*/}*^{Δ},* and *Cbl-b*^{*C373A*/*C373A*} mice were infected intravenously with *Candida albicans* (10⁵ CFU/21.5g body weight) and monitored for the indicated time periods. Plots depict **(a)** weight loss over time after infection as compared to starting weight (p values assessed by two-way ANOVA) or **(b)** survival over time after infection (p values assessed with log rank test). n = 6 for Cbl-b^{+/+} and Cbl-b^{C373A/C373A}, n = 7 for Cbl-b^{Δ/Δ} mice. **(c)** *Candida albicans* fungal load in depicted organs at day 7 after infection, plotted as colony forming units (CFU) per gram organ weight. Each dot represents an individual mouse. **(d,e)** Representative kidney sections from mice 7 days after systemic *Candida albicans* infection, stained with periodic-acid-Schiff (PAS) **(d).** Insets show regions of renal inflammation from boxed areas at increased magnification. **(e)** Combined inflammatory score based on the extent of renal immune cell infiltration, inflammation and tissue destruction. **(f,g)** Representative kidney sections from mice 7 days after *Candida albicans* infection, stained with Gomori methenamine silver (GMS) **(f).** Insets show regions of fungal growth from boxed areas at increased magnification. Yellow arrowhead indicates fungal hyphae. **(g)** Plot depicts intralesional fungal load, as scored by histopathologic assessment of GMS stained kidney sections. **(h,i)** Representative kidney sections stained for the neutrophil marker Ly-6G **(h).** Insets show regions of neutrophil infiltration from boxed areas. **(i)** Plot depicts the percentage of kidney area scored positive for Ly-6G staining using Definiens Tissue Studio software. For panels **(d-g)** n = 4 for Cbl-b^{+/+}, and n = 5 for Cbl-b^{Δ/Δ} mice; for panels **(h,i)** n = 4 for Cbl-b^{+/+}, n = 4 for Cbl-b^{Δ/Δ} mice; for panels **(d-i)** 4 sections per mouse were analyzed. For panels **(c,e,g,i)** data are shown as means ± standard deviation. For all experiments, 1 representative experiment of 3-6 independent repeats is shown. * P <0.05, ** P < 0.01, *** P <0.001 as calculated with *Student's t-test,* unless stated otherwise.

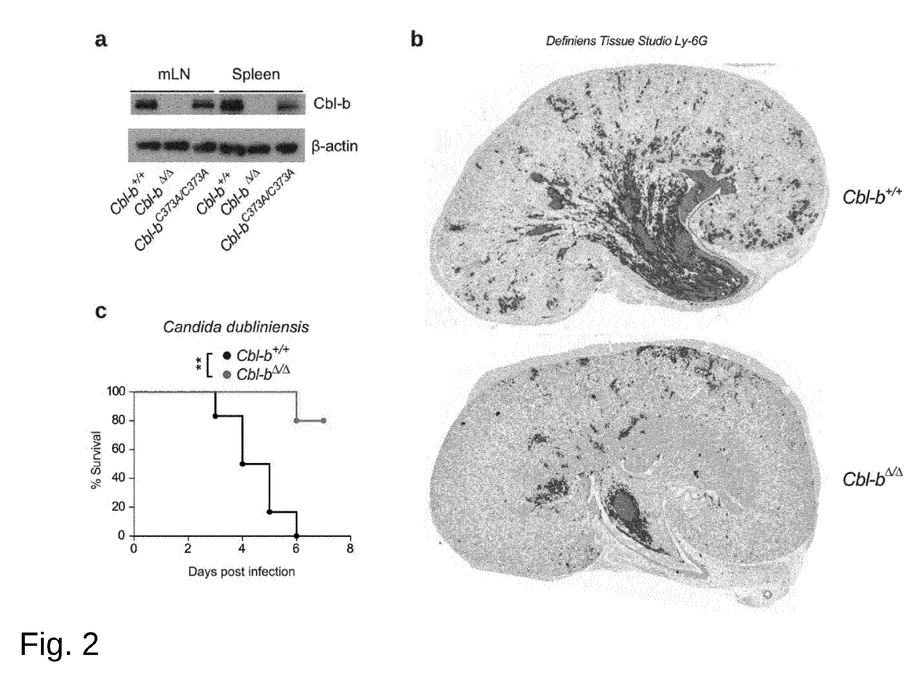

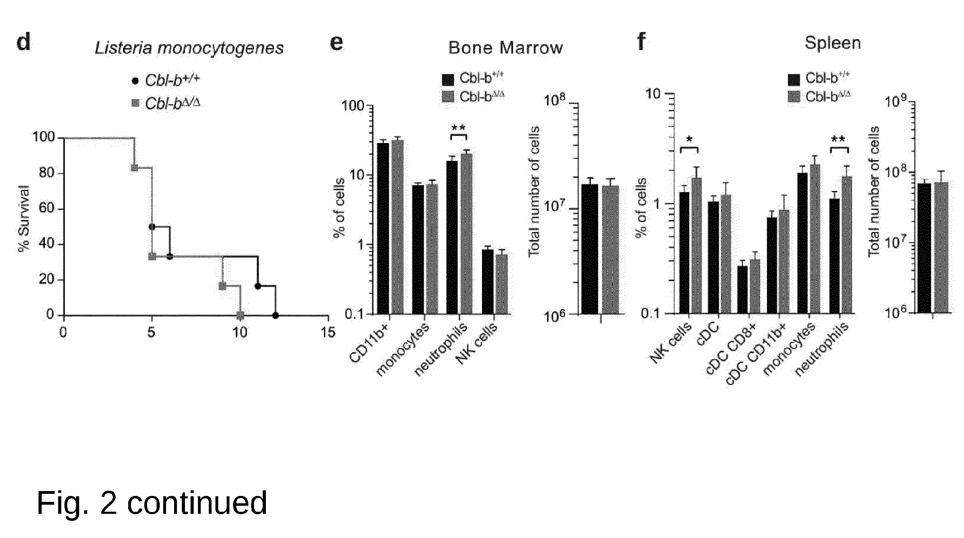

**Fig. 2****: Candida ssp. and Listeria monocytogenes infection.**
   **(a)** Protein levels of Cbl-b in lysates prepared from mesenteric lymph nodes (mLN) or spleens of mice of the indicated genotypes as assessed with Western blotting. β-Actin is shown as loading control. **(b)** Representative Definiens Tissue Studio software analysis of Ly-6G stained kidney sections from Figure 1h. **(c)** *Cbl-b+*/*+* and *Cbl-b,b*Δ/Δ mice were infected intravenously with *C*. *dubliniensis* (10⁶ CFUs)and monitored for the indicated period of time. Plot depicts survival over time after infection (*P* values assessed with log rank test) *n* = 5 for *Cbl-b+*/*+* and *n* = 6 for *Cbl-bΔ*/*Δ* cohort. **(d)** *Cbl-b+*/*+* and *Cbl-bΔ*/*Δ* mice were infected intravenously with *Listeria monocytogenes* (10⁵ CFUs)and monitored for the indicated period of time. Plot depicts survival over time after infection **(e,f)** Frequency of indicated innate immune cell lineages and total immune cell counts of **(e)** femurs or **(f)** spleens of mice of the indicated genotypes as assessed by flow cytometry. n = 8 for *Cbl-b*+/+, n = 11 for *Cbl-b*Δ/Δ littermates. Data are shown as means ± standard deviation. For panels **(a,b,d,e,f)** 1 representative of 3, for panel **(c)** 1 representative of 2 independent experiments is shown. * P <0.05, ** P < 0.01 as calculated with *Student's t-test,* unless stated otherwise.
**Fig. 3****: Cb1-b in immune cells protects against fungal pathology.**
   **(a,b)** *Cbl-b*^{*+*/*+*} mice were reconstituted with bone marrow from *Cbl-b*^{*+*/}*⁺ , Cbl-b*^{*Δ*/}*^{Δ},* or *Cbl-b*^{*C373A*/*C373A*} mice after lethal irradiation. Eight weeks after reconstitution, mice were infected intravenously with *Candida albicans* (10⁵ CFU/21.5 g body weight) and monitored for the indicated time periods. Plots depict **(a)** weight loss over time after infection, as compared to starting weight (P values assessed by two-way ANOVA) or **(b)** survival over time after infection (P values assessed with log rank test). n = 6 for all cohorts. **(c,d)** *Cbl-b*^{+/+}/*Rag2*^{*Δ*/*Δ*} or *Cbl-b*^{*Δ*/}*^{Δ}Rag2*^{*Δ*/*Δ*} mice were infected and scored as in **(a)** for **(c)** weight loss and **(d)** mortality. n = 7 for *Cbl-b*^{*+*/*+*}/*Rag2*^{*Δ*/}*^{Δ},* n = 6 for *Cbl-b*^{*Δ*/*Δ*}/*Rag*^{*2Δ*/*Δ*} mice. **(e,f)** *Rag2*^{*Δ*/*Δ*} mice were reconstituted with bone marrow from *Cbl-b*^{*+*/*+*}/*Rag2*^{*Δ*/*Δ*} or *Cbl-b*^{*Δ*/*Δ*}/*Rag*^{*2Δ*/*Δ*} mice after lethal irradiation. Eight weeks after reconstitution mice were infected and scored as in **(a)** for **(e)** weight loss and **(f)** mortality. n = 6 for *Cbl-b*^{*+*/*+*}/*Rag2*^{*Δ*/*Δ*} into *Rag2*^{*Δ*/}*^{Δ},* n = 5 for *Cbl-b*^{*Δ*/*Δ*}/*Rag*^{*2Δ*/*Δ*} into *Rag2*^{*Δ*/*Δ*} mice. **(g,h)** *Cbl-b*^{*+*/*+*}, *Cbl-b*^{*Δ*/}*^{Δ}, Cbl-b*^{*+*/*+*}/*Rag2*^{*Δ*/}*^{Δ},* or *Cbl-b*^{*Δ*/*Δ*}/*Rag*^{*2Δ*/*Δ*} mice were infected on their shaved dorsum with 2 x 10⁸ CFU *Candida albicans.* Plot depicts fungal load in the skin 3 days after infection, plotted as CFU/total infected skin area. Dots represent individual mice. For panels **(a,b,e,f,g,h)** 1 representative of 3, for panels **(c,d)** 1 representative of 4 independent experiments is shown. * P <0.05, ** P < 0.01, *** P <0.001 as calculated with *Student's t-test,* unless stated otherwise.

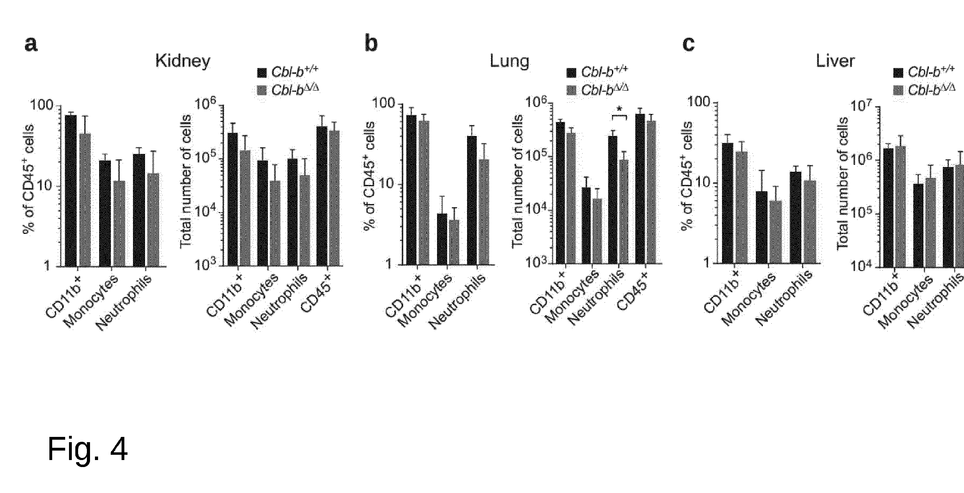

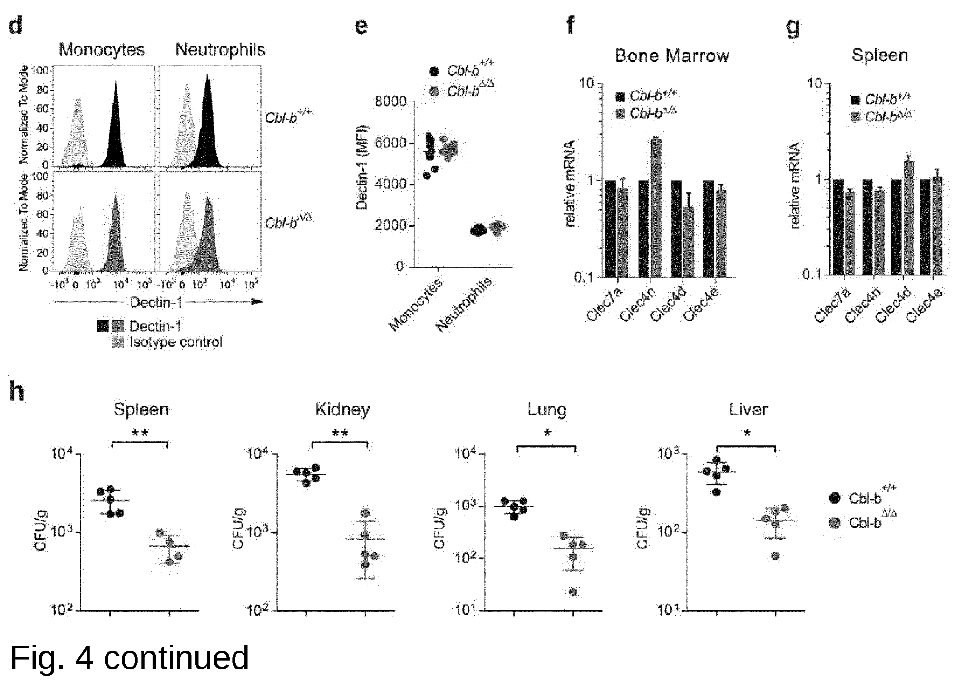

**Fig. 4****: Immune infiltrations, CLR expression, and early fungal loads.**
   (a,b,c) Cellular recruitment of the indicated immune cell lineages to kidneys **(a),** lungs **(b),** and livers **(c)** of *Cbl*-*b*+/+ and *Cbl-b*_{*Δ*/*Δ*} mice 24h after infection with *Candida albicans* (10₅ CFU/21.5g body weight). Plots depict percentages of CD45+ hematopoietic cells (left panels) or total numbers of recruited cells per organ (right panels). n = 5 for *Cbl-b*_{*+*/*+*}, n = 6 for *Cbl-b*_{*Δ*/*Δ*} mice. **(d,e)** Dectin-1 protein expression on peripheral blood monocytes and neutrophils isolated from *Cbl-b+*/*+* and *Cbl-b*_{*Δ*/*Δ*} mice as assessed by blow cytometry. **(d)** Representative stainings and **(e)** Dectin-1 mean fluorescence intensities (MFI) are shown. Dots in **(e)** represent individual mice. **(f,g)** mRNA expression levels of the indicated C-type lectin receptors in **(f)** bone marrow cells and **(g)** splenocytes of *Cbl*-*b*+/+ and *Cbl-b*_{*Δ*/*Δ*} mice as assessed by quantitative PCR. Experiments were done in triplicates. **(h)** *Candida albicans* fungal load in depicted organs 24h after infection, plotted as colony forming units (CFU)/gram organ weight. Each dot represents an individual mouse. For panels **(a,b,c,e,f,g,h)** data are shown as means ± standard deviation. For panels **(a,b,c,f,g)** 1 representative of 3, for panels **(d,e)** 1 representative of 5 independent experiments is shown. * P <0.05, ** P < 0.01 as calculated with *Student's t-test.*

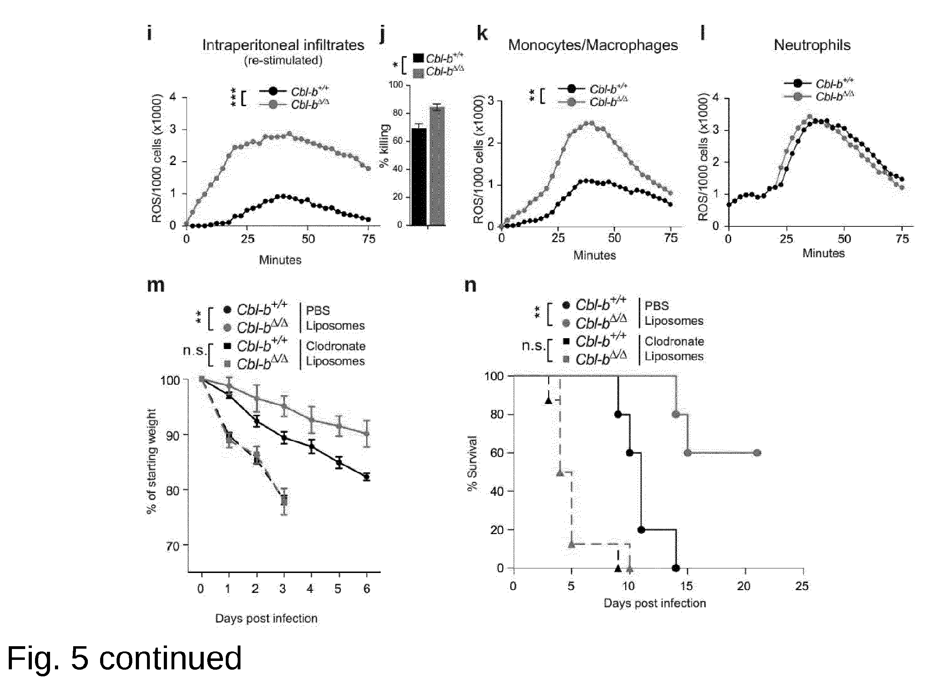

**Fig. 5****: Cb1-b controls anti-fungal activities of dendritic cells and macrophages.**
   **(a-e)** Killing capacity of BM-neutrophils **(a),** BM-M **(b),** BM-DC **(c),** BM-monocytes **(d),** or splenic DC **(e)** as assessed by co-culture with *Candida albicans.* Assays performed in quadruplicates. **(f,g)** Killing capacity of BM-DC in the presence of **(f)** the phagocytosis inhibitor Dynasore or an anti-Dectin-1 antibody, or **(g)** the Syk inhibitor R406. **(h,i)** ROS production by intraperitoneal immune infiltrates of *Cbl*-*b*^{+/+} or *Cbl-b*^{*Δ*/*Δ*} mice 24h after intraperitoneal *Candida albicans* infection (5 * 10⁶ CFU/21.5 g body weight). Plots depict **(h)** ROS production by immune infiltrates without re-stimulation or **(i)** after re-stimulation with *Candida albicans.* Experiments performed in triplicates. **(j)** Killing capacity of immune infiltrates from **(h,i). (k,l)** ROS production of **(k)** monocytes/macrophages or **(1)** neutrophils FACS sorted from infiltrates in **(h,i). (m,n)** *Cbl*-*b*^{+/+} or *Cbl-b*^{*Δ*/*Δ*} mice were injected with PBS liposomes or clodronate liposomes 24h before and 24h after intravenous infection with *Candida albicans* (10⁵ CFU/21.5g body weight) and monitored for **(m)** weight loss, as compared to starting weight (P values assessed by two-way ANOVA), or **(n)** survival (P values assessed with log rank test), n = 5 for PBS liposome treated, n = 8 for clodronate liposome treated cohorts. For panels **(a-g, j, m)** data are shown as means ± standard deviation. For panels **(a-f)** 1 representative of 5, for panels **(g-n)** 1 representative of 3 independent experiments is shown. * P <0.05, ** P < 0.01, *** P <0.001 as calculated with *Student's t-test,* unless stated otherwise.

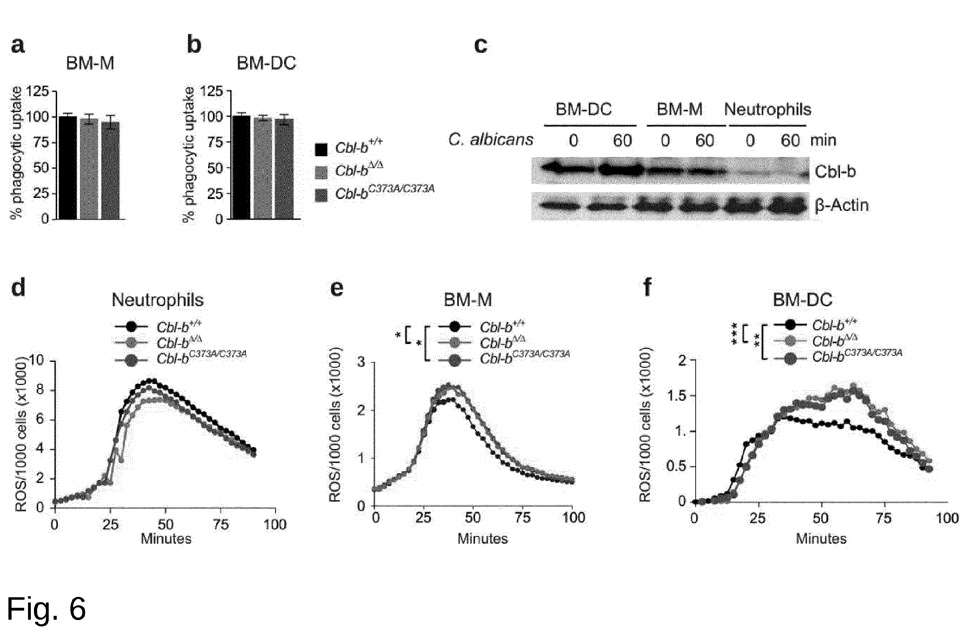

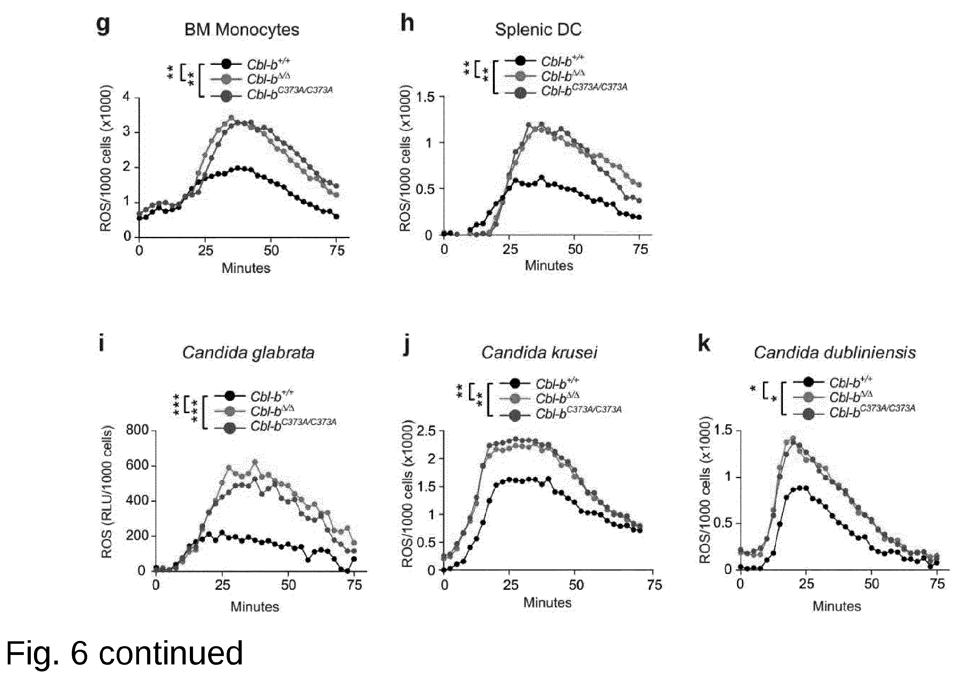

**Fig. 6****: Phagocytosis and ROS production in response to fungal stimulation.**
   **(a,b)** Rate of phagocytosis by **(a)** BM-M or **(b)** BM-DC of the indicated genotypes co-cultured with *Candida albicans.* **(c)** Cbl-b protein expression as assessed by Western blotting from lysates prepared from C. *albicans* stimulated or unstimulated BM-DC, BM-M or bone marrow neutrophils derived from *Cbl-b*^{*+*/*+*} mice. β-Actin blots are shown as loading control. **(d-h)** Reactive oxygen species (ROS) production by **(d)** bone marrow neutrophils, **(e)** BM-M, **(f)** BM-DC, **(g)** bone marrow monocytes, or **(h)** splenic DC co-cultured with *Candida albicans* and monitored in real time over the indicated time periods using the luminol assay. Experiments were performed in triplicates. Values are expressed as relative light units per 1.000 cells. **(i-k)** ROS production by BM-DC of the indicated genotypes stimulated with **(i)** *C. glabrata,* **(j)** *C*. *krusei,* or **(k)** *C. dubliniensis,* monitored and analyzed as in **(d-h).** Data in panels **(a,b)** are shown as means ± standard deviation. P values for panel **(a,b)** were calculated with *Student's t-test,* for panels **(d-k)** with two-way ANOVA. For all experiments 1 representative of 3 independent experiments is shown. * P < 0.05, ** P < 0.01, *** P <0.001.

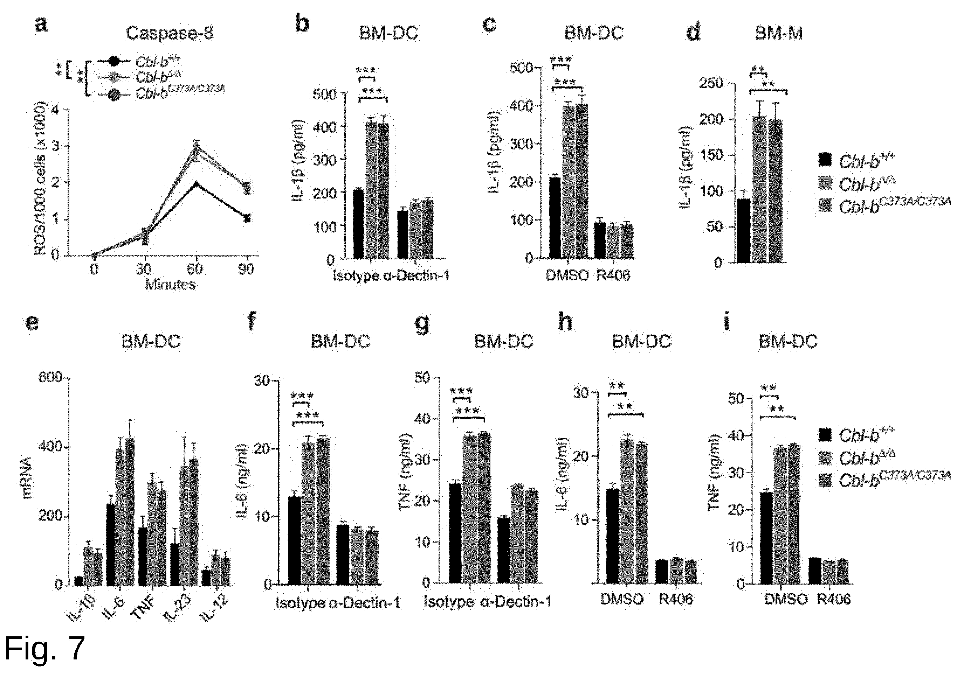

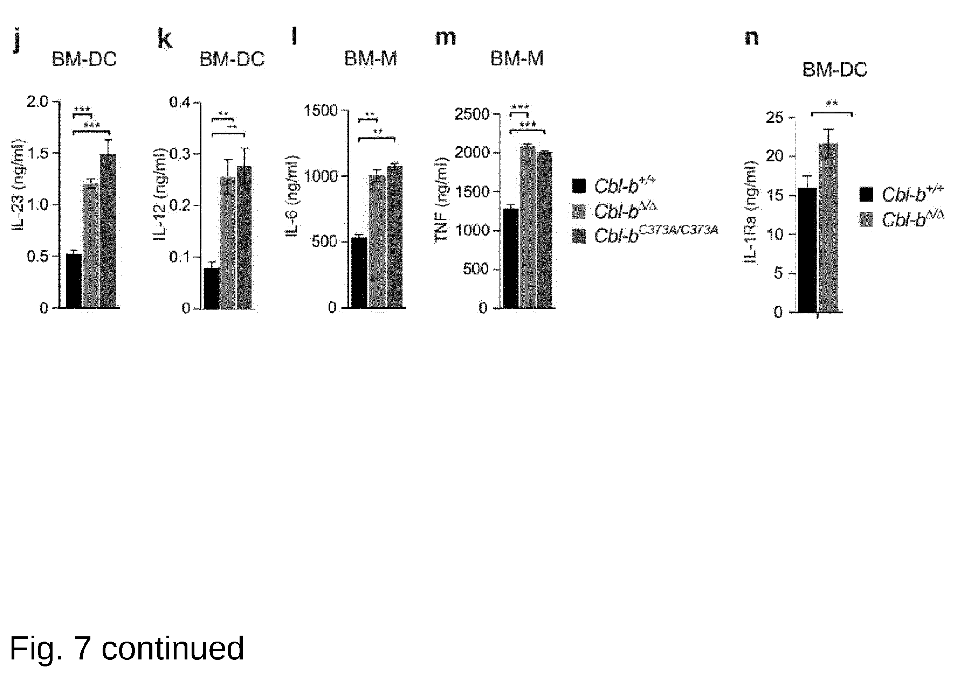

**Fig. 7****: Cytokine release in response to fungal stimulation.**
   **(a)** Activation of Caspase-8 in BM-DC of the indicated genotypes monitored at the indicated time points after stimulation with *C*. *albicans,* using the CaspGLOW assay (*P* values assessed by two-way ANOVA). **(b,c)** Secretion of IL-1β by BM-DC of the indicated genotypes stimulated with *C*. *albicans* for 18h in the presence of **(b)** an anti-Dectin-1, or isotype control antibody, or (c) the SYK inhibitor R406, or DMSO as assessed by ELISA. **(d)** Secretion of IL-1β by BM-M upon 18 h co-culture with *C. albicans* as assessed with ELISA. **(e)** Induction of cytokine RNA expression in BM-DC of the indicated genotypes stimulated for 2 h with *C*. *albicans,* as assessed by quantitative PCR. Experiments were done in triplicates. **(f-i)** Secretion of the indicated cytokines by BM-DC of the indicated genotypes upon 18 h co-culture with *C*. *albicans* in the presence of **(f,g)** an anti-Dectin-1, or isotype control antibody, or **(h,i)** the SYK inhibitor R406, or DMSO as assessed with ELISA. **(j-n)** Secretion of the indicated cytokines by **(j,k,n)** BM-DC, or **(l,m)** BM-M of the indicated genotypes upon 18 h co-culture with C. *albicans* as assessed with ELISA. Data in all panels are shown as means ± standard deviation. *P* values for all panels except **(a)** were calculated with *Student's t-test,* for panel **(a)** with two-way ANOVA. For panels **(a-d,f-n)** 1 representative of 4, for panels **(e)** 1 representative of 5 independent experiments is shown. ** *P* < 0.01, *** *P* < 0.001.

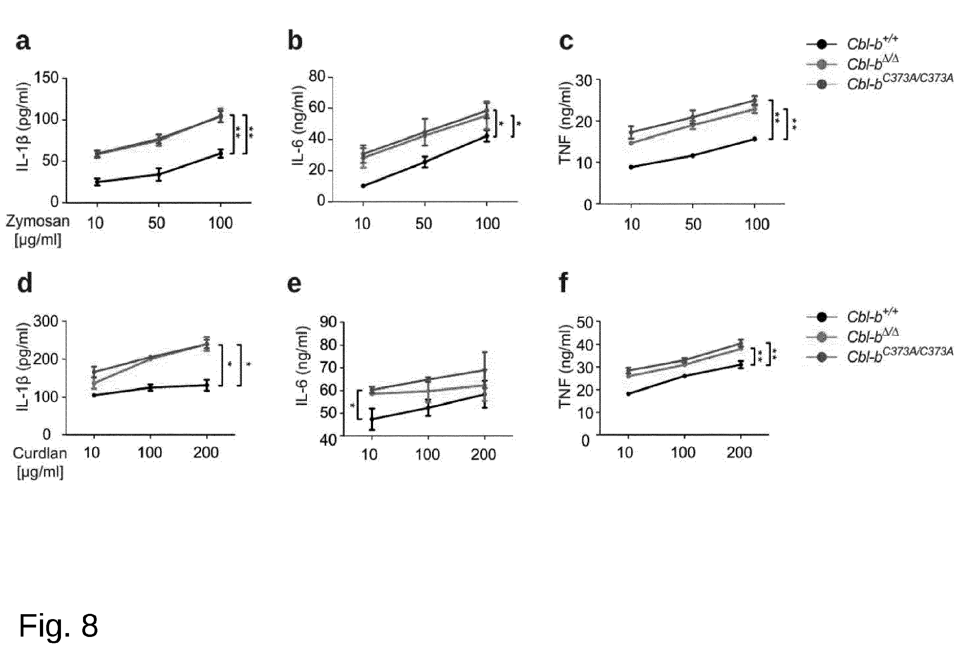

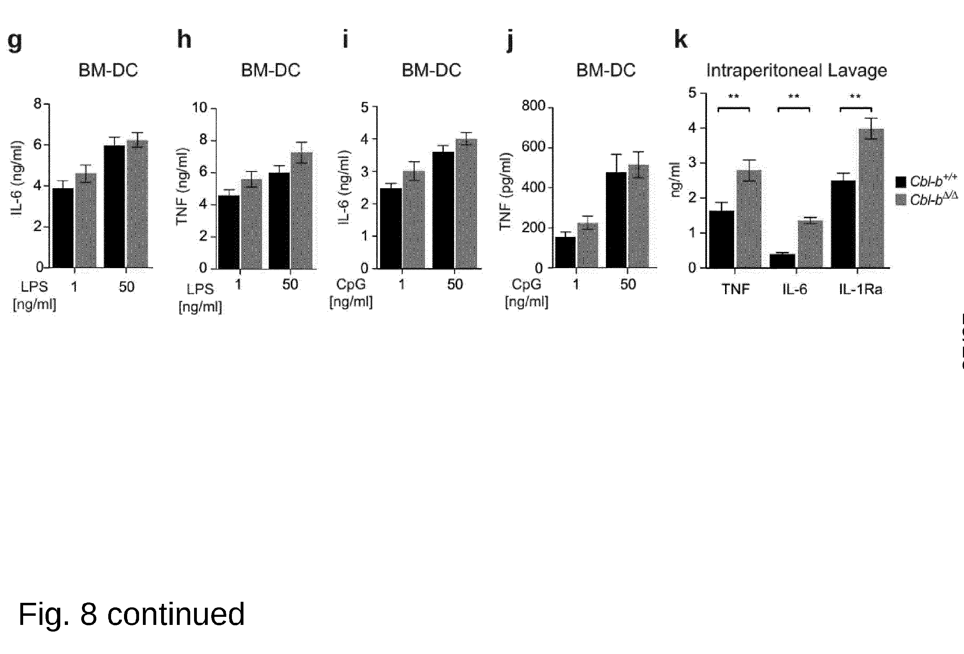

**Fig. 8****: Zymosan, Curdlan, LPS, CpG, and *C*. *albicans* triggered cytokine release by BM-DC and intraperitoneal inflammatory infiltrates.**
   **(a-f)** Secretion of the indicated cytokines by *Cbl-b*^{*+*/}*⁺, Cbl-bC373A*/*C373A* or *Cbl-bΔ*/*Δ* BM-DC stimulated with increasing doses of either Zymosan **(a-c),** or Curdlan **(d-f)** for 18h, as assessed by ELISA. **(g-j)** Secretion of the indicated cytokines by *Cblb*^{+/+} or *Cblb*^{*Δ*/*Δ*} BM-DC stimulated with the indicated doses of either LPS **(g,h),** or CpG **(i,j)** for 18 h, as assessed by ELISA. **(k)** Expression of the indicated cytokines in the intraperitoneal lavages of *Cblb*^{+/+} or *Cblb*^{*Δ*/*Δ*} mice 24h after intraperitoneal infection with *C*. *albicans* as in Figure 5h-i as assessed by ELISA. Data in all panels are shown as means ± standard deviation. *P* values as calculated with **(a-f)** two-way ANOVA, or **(k)** *Student's t-test.* For all panels 1 representative of 3 independent experiments is shown. * *P* < 0.05, ** *P* < 0.01
**Fig. 9****: Increased CLR mediated signaling in the absence of Cbl-b.**
   **(a)** *Cbl*-*b*^{+/+}, *Cbl-b*^{*Δ*/*Δ*} or *Cbl-b*^{*C373A*/*C373A*} BM-DC were stimulated with *Candida albicans* for the indicated periods of time and analyzed by Western blotting for levels of phosphorylated tyrosine. **(b)** BM-DC of the indicated genoytpes were stimulated as in **(a)** and analyzed for phosphorylated or total amounts of the indicated signaling molecules. **(c)** *Cbl-b*^{*+*/*+*} and *Cbl-b*^{*Δ*/*Δ*} BM-DC were stimulated for 90min with Trehalos-6,6-dimycolate (TDM), heat killed *Candida albicans* (HK), or *Candida albicans* hyphae (Hyph), and lysates were analyzed for levels of phosphorylated Src, phosphorylated Shp-2, and phosphorylated Syk by Western blotting. β-Actin is shown as a loading control. **(d)** BM-DC of the indicated genoytpes were stimulated as in **(a)** and analyzed for phosphorylated or total amounts of the indicated molecules. (e,f) *Cbl-b*^{*+*/*+*} or *Cbl-b*^{*Δ*/*Δ*} BM-DC were stimulated with *Candida albicans* for 90min or left unstimulated, fixed with 4% paraformaldehyde, stained for **(e)** phosphorylated Shp-2 or **(f)** phosphorylated Syk, and analyzed by confocal microcopy. Representative images for each genotype and time point are shown. Arrowheads depict phagosome localized **(e)** phosphorylated Shp-2 or **(f)** phosphorylated Syk. Cells were counterstained with DAPI (blue). For all panels, 1 representative of 4 independent experiments is shown.

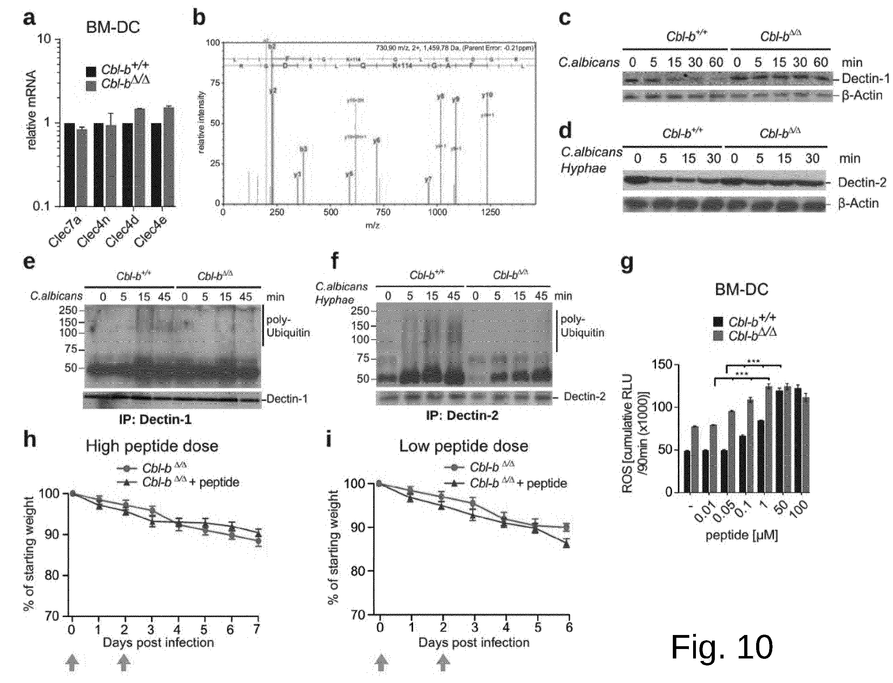

**Fig. 10****: SYK and Dectin-1, -2 ubiquitination and CBL-B inhibitory peptide treatment.**
   **(a)** mRNA expression levels of the indicated C-type lectin receptors in *Cblb*^{*+*/*+*} and *Cblb*^{*Δ*/*Δ*} BM-DC assessed by qPCR. Experiments were done in triplicates. **(b)** Mass spectrometric analysis of the ubiquitin chains generated by *in vitro* ubiquitination by CBL-B. A representative MS/MS spectrum acquired for a peptide derived from K48-linked Ubiquitin chains, using a sample processed identically and in parallel to the sample shown in Fig. 11a, is shown. Both β-type (red) and γ-type (blue) ions of the selected peptide are indicated. **(c,d)** Immunoblot analysis of **(c)** Dectin-1 and **(d)** Dectin-2 protein expression in *Cblb*^{+/+} and *Cblb*^{*Δ*/*Δ*} BM-M upon infection with **(c)** *C*. *albicans* or **(d)** *C. albicans* hyphae with an anti-Dectin-1 or anti-Dectin-2 antibody, respectively. β-Actin blots are shown as loading control. **(e,f)** Immunoblot analysis of **(e)** Dectin-1 and **(f)** Dectin-2 ubiquitination in E64-pre-treated *Cblb*^{+/+} and *Cblb*^{*Δ*/*Δ*} BM-M after immunoprecipitation with an anti-Dectin-1 or anti-Dectin-2 antibody, respectively, with an anti-ubiquitin antibody. Dectin-1 and Dectin-2 immunoblots are shown as controls. **(g)** ROS production by *Cblb^{+l+}* and *Cblb*^{*Δ*/*Δ*} BM-DC co-cultured with *C*. *albicans* in the presence of the indicated concentrations of the TKB binding peptide. Plot depicts cumulative ROS production over 75 minutes for the indicated peptide concentrations. Experiments were performed in triplicates. Values are expressed as relative light units per 1.000 cells. **(h,i)** *Cblb*^{*Δ*/*Δ*} mice were infected intravenously with *C*. *albicans* (10⁵ CFU/21.5g body weight), injected intraperitoneally with **(h)** 100 µg or **(i)** 20 µg of the TKB binding peptide at day 0 and day 2 after infection, and monitored for weight loss over time after infection as compared to starting weight (*P* values assessed by two-way ANOVA). *n* = 5 for treated and untreated cohorts. **(j,k)** Representative kidney sections from *Cblb*^{+/+} mice treated as in Fig. 12g, and stained with **(j)** Gomori methenamine silver (GMS) or **(k)** for the neutrophil marker Ly-6G. Yellow arrowhead in **(j)** indicates fungal hyphae. *n* = 3 for peptide treated and untreated cohorts. 4 kidney sections per mouse were analyzed. Data in **(a,g)** are shown as means ± standard deviation. For panel **(a,b)** 1 representative of 4, for panel **(c-k)** 1 representative of 3 independent experiments is shown. *** *P* < 0.001 as calculated with *Student's t-test.*
**Fig. 11****: Cb1-b ubiquitinates Syk.**
   **(a)** *In vitro* ubiquitination of active SYK by recombinant Cbl-b, Cbl-b²⁹⁻⁴⁸³, or C373A Cbl-b²⁹⁻⁴⁸³. Assays analyzed by Western blotting for Syk (upper panel) or Ubiquitin (lower panel). Note, that Cbl-b forms non substrate attached poly-ubiquitin chains. * denotes unspecific bands. **(b)** *In vitro* ubiquitination of inactive Syk (Syk-GST) or active Syk by Cbl-b²⁹⁻⁴⁸³. Control, no ATP. Assays were analyzed as in **(a). (c)** Lysates from BM-DC stimulated with *Candida albicans* immunoprecipitated with an anti-Cbl-b antibody or IgG control. Precipitates were probed for Cbl-b and Syk. Aliquots were blotted for Cbl-b and Syk as a loading control. **(d)** BM-DC were stimulated with *Candida albicans* in the presence or absence of MG132 (10µM) and analyzed by Western blotting. **(e)** Sequence of the TKB binding peptide. Antennapedia homeodomain derived sequence and Syk derived sequences are indicated. Tyrosine 317 is shown in red, p in black circle symbolizes phosphorylation of Tyrosine 317. **(f)** *In vitro* ubiquitination of active recombinant Syk by Cbl-b²⁹⁻⁴⁸³ in the presence or absence of 100µM TKB binding peptide. Control, no ATP. Assays were analyzed by Western blotting for Syk (upper panels) or ubiquitin (lower panels). **(g)** BM-DC of the indicated genotypes were stimulated with *Candida albicans* in the absence or presence of 100µM TKB binding peptide and monitored for ROS production in real time, using the luminol assay. Experiments were performed in triplicates. Values are expressed as relative light units per 1000 cells (P values calculated with two-way ANOVA). **(h)** Killing capacity of BM-DC of the indicated genotypes as assessed by 24h co-culture with *Candida albicans* in the presence or absence of 100µM TKB binding peptide. Assays were performed in quadruplicates. Data are shown as means ± standard deviation. P values were calculated with *Student's t-test.* For all panels, 1 representative of 3 independent experiments is shown ** P < 0.01, *** P <0.001.
**Fig. 12****: Cb1-b inhibition enhances anti-fungal immunity.**
   **(a,b) *Cbl*-*b*^{+/+}** mice were infected intravenously with *Candida albicans* (10⁵ CFU/21.5g body weight), and injected intraperitoneally with 100 µg TKB binding peptide twice. Plots depict **(a)** weight loss (P values assessed by two-way ANOVA) and **(b)** survival (P values assessed with log rank test). **(c)** Blood urea concentration 7 days after infection. N = 5 for all cohorts. (c,d,e) *Cbl*-*b*^{+/+} mice were treated as in **(a-c)** with 20 µg peptide. Plots depict **(d)** weight loss (P values assessed by two-way ANOVA; n = 5 for all cohorts), **(e)** Blood urea concentration and **(f)** kidney fungal load 7 days after infection. **(g,h,i)** *Cbl*-*b*^{+/+} mice infected as in **(a-c),** were injected intraperitoneally with 100 µg of peptide twice. Plots depict **(g)** weight loss (P values assessed by two-way ANOVA), **(h)** kidney fungal load 48h after the second peptide treatment, and **(i)** survival (P values assessed with log rank test). **(a,d,g)** Blue arrows indicate time points of peptide treatment. **(j)** PAS stained kidney sections from mice treated as in **(g)** and sacrificed 2 days after the second peptide treatment. Boxed areas shown at increased magnification. **(k)** Inflammatory score of infected kidneys from **(j).** n = 3 for both cohorts. 4 sections per mouse were analyzed. **(a,b,c,d,e,f,g,h,i)** Data shown as means ± standard deviation. Dots represent individual mice. For panels **(a-c),** 1 representative of 5, for panel **(c-j)** 1 representative of 3 independent experiments is shown. ** P < 0.01, *** P <0.001 as calculated with *Student's t-test.*

### Examples

### Example 1 - Materials & methods

Mice: *Cbl-b*^{*Δ*/}*^{Δ}, Cbl-b*^{*C373A*/*C373A*} and *Rag2*^{*Δ*/*Δ*} deficient mice have been described previously and were bred on a C57BI/6 background. Mouse genotypes were assessed by PCR. Of note, only age- and sex-matched littermates from respective breedings were used for experiments. All mice were bred and maintained in accordance with ethical animal license protocols complying with the current Austrian law.

Generation of bone marrow chimeric mice: To generate bone marrow chimeric mice, 8 week old wild type or *Rag2*^{*Δ*/*Δ*} deficient C57BL6 mice were split dose irradiated (2 x 6 Gy) and reconstituted 18h after the second irradiation with 3 million donor cells by intravenous injection. Experiments were carried out 8 weeks later to allow for full reconstitution. Reconstitution efficiency under the applied conditions was tested by using congenic CD45.1 recipient mice and staining of splenocytes for CD45.1 (recipient derived) and CD45.2 (donor derived), 8 weeks after reconstitution, and was routinely >98%.

Isolation and differentiation of neutrophils, monocytes, and dendritic cells: Neutrophils were isolated from tibias and femurs of mice using a Percoll-gradient (GE Healthcare) as previously described (Boxio et al., 2004) or were purified using MACS technology (Miltenyi) after Ly-6G labeling. Bone marrow monocytes were purified by FACS sorting after labeling with antibodies specific for CD11b, Ly-6G, and Ly-6C. Splenic dendritic cells were FACS sorted after labeling with antibodies to CD19, TCRβ, Ly-6G, and CD11c. Purified neutrophils, monocytes, and splenic dendritic cells were cultured in RPMI-1640 medium (PAA). BM-DC or BM-M were obtained by differentiation from bone marrow precursor cells. Peripheral blood monocytes or neutrophils were obtained by sub-mandibular bleeding and collected in potassium EDTA tubes (Sarstedt, Germany). Red blood cells were lysed using an Ammonium-Chloride-Potassium (ACK) buffer.

Flow cytometry: Antibody labeling of leukocytes was carried out in FACS staining buffer (PBS supplemented with 2% FBS and 2mM EDTA) on ice for 30min after blocking of Fc-receptors. Cell viability was assessed with the Fixable Viability Dye eFluor780 (eBioscience). Immune cell recruitment was assessed by Collagenase 4 (Worthington)/DNAse (Roche) digestion of organs, viability staining, Fc-blocking, antibody staining, and subsequent flow cytometric analysis. Samples were acquired with a BD LSRII flow cytometer and data were analyzed using FlowJo software (Treestar).

Measurements of cytokine release, ROS production, Caspase-8 activity: Cytokine secretion by cell culture supernatants or in the peritoneal lavage was assessed with ELISA kits purchased from eBioscience (IL-1β, TNF, IL-6, IL-12, IL-23) or R&D Systems (IL-1Ra) according to the manufacturer's instructions. Production of reactive oxygen species (ROS) was measured with as assay using luminol as the probe in real-time over 90min as described previously (Bourgeois et al., 2009). Caspase-8 activity was assessed using the CaspGLOW assay (Promega) according to the manufacturer's instructions.

Phagocytosis assay: For phagocytosis assays *Candida albicans* (strain SC5314) were Alexa 488 labeled in 100mM HEPES buffer (pH 7.5). BM-DC or BM-M were co-cultured with labeled *Candida albicans* at 37°C for 45 minutes. Fluorescence by fungal cells adherent to, but not phagocytosed by, phagocytes, were quenched with trypan blue (Sigma Aldrich), and the rate of phagocytosis was assessed by flow cytometry.

Killing assay: To assess the fungicidal activity, the indicated immune cells were plated in replicates at a density of 1 x 10⁵ cells/well in 96-well plates. Cells were incubated with *Candida albicans* at a multiplicity of infection of 1:500 for 24h. After incubation, phagocytes were fixed by the addition of paraformaldehyde at a final concentration of 4%. Subsequently, *Candida albicans* were stained with Crystal Violet (Sigma) and killing was assessed by a comparison of colony numbers in wells with or without phagocytes.

Mouse infection and clodronate liposome treatment: 8-10 week old mice were infected with 10⁵ colony forming units (CFU) of C. *albicans* (strain SC5314), 10⁶ colony forming units (CFU) of C. *dubliniensis,* or 10⁵ CFUs of *Listeria monocytogenes* (strain EGD) intravenously. *C. albicans* CFUs were assessed from various organs as described (Wirnsberger et al., 2014). For clodronate or PBS liposome treatment experiments, mice were injected intraperitoneally with 100ul/10g mouse weight of liposomes both 24h before and 24h after intravenous *C. albicans* infections. Clodronate or PBS control liposomes were obtained from clodronateliposomes.org, and handled according to the manufacturer's instructions. All animal infection experiments were evaluated by the ethics committee of the Medical University of Vienna and approved by the Federal Ministry of Science and Research, Vienna, Austria.

BM-M and BM-DC stimulation experiments: BM-M and BM-DC were stimulated with *C. albicans* (strain SC5314), Zymosan (Invivogen), Curdlan (Invivogen), TDM (Invivogen), CpG (Invivogen), LPS (Invivogen), or *C. albicans* hyphae. For inhibition experiments cells were treated with the Syk inhibitor R406 (Selleckchem), anti-Dectin-1 antibody (Invivogen), MG132 (Calbiochem), or Dynasore (Sigma-Aldrich), 30min prior to stimulation (MOI: Immune cell: *C*. *albicans* = 1:2). Fungal isolates of *Candida krusei, Candida glabrata,* and *Candida dubliniensis* were kindly provided by the Allgemeines Krankenhaus Vienna (AKH). Fungal isoates were tested by PCR to verify the respective species.

Quantitative PCR: mRNA was isolated using the SV-Total RNA Isolation System (Promega) or Rneasy Kit (Qiagen), reverse transcribed using the Reverse Transcription System (Promega) or the iScript cDNA synthesis Kit (Biorad). QPCR was performed with KAPA SYBR Fast Universal (Peqlab).

Western blotting and Immunoprecipitation: Western blotting was performed according to standard protocols. Blots were blocked for 1 hour with 5% bovine serum albumin in 1 x TBS/0.1% Tween-20, or 4% BSA in 1 x TBS/0.1% Tween-20 for phosphorylated epitopes, followed by overnight incubation at 4°C with primary antibodies. Subsequently, blots were washed three times in 1 x TBS/0.1% Tween-20 for 15 min, followed by incubation with HRP-conjugated secondary antibodies (1:2500; GE Healthcare # NA9340V) for 45 min at room temperature, washed three times in 1 x TBS/0.1% Tween-20 for 15 min and visualized using enhanced chemiluminescence (ECL Plus, Pierce). Anti-β-Actin mAbs were used to control for protein loading. For Cbl-b-immunoprecipitation experiments, cells were stimulated with *C. albicans,* lysed in RIPA buffer (Cell Signaling Technology) containing phosphatase and proteinase inhibitors (Halt Protease and Phosphatase inhibitor Cocktail; Thermo Scientific), pre-cleared with Protein A/G Plus Agarose beads (Santa Cruz Biotechnology), and incubated overnight with an anti-Cbl-b antibody. Precipitates were analyzed by Western blotting using the indicated antibodies. For Dectin-1 and Dectin-2 immunoprecipitates, BMM of the indicated genotypes were pretreated with E-64 (10µM) for 30 min, infected with *C. albicans* yeast or hyphae for the indicated periods of time, lysed in RIPA buffer containing 2% SDS on ice for 30min, and diluted with RIPA buffer without SDS for a final SDS concentration of 0.4%. After brief sonication, lysates were immunoprecipitated with an anti-Dectin-1 or anti-Dectin-2 antibody at a concentration of 1µg/ml. Precipitates were analyzed by Western blotting with the indicated antibodies.

*In vitro* ubiquitination assays: Reaction mixtures for *in vitro* ubiquitination assays contained 100nm Ube1 (purified in-house), 500Nm UbcH5b (Enzo Life Sciences), 150nm full length human Cbl-b (Abnova) or human Cbl-b²⁹⁻⁴⁸³, or a catalytically inactive human C373A Cbl-b29-483 mutant, and 100mM ubiquitin (Sigma-Aldich) with 300 nm of substrate in reaction buffer (20mM Tris-Hcl pH 7.5, 150mM NaCl, 10mM MgCl₂ and 1mM DTT). Substrates were active (phosphorylated) human Syk (Merck Millipore) and inactive (non-phosphorylated) human Syk-GST (Life technologies). 1MM ATP was added to initiate the reaction and incubated at 37°C for 30 min. For TKB binding peptide mediated Cbl-b inhibition, Cbl-b was pre-incubated with the TKB binding peptide at a concentration of 100µM for 20 min at 24°C, before addition to the reaction mixture. Assays were resolved by SDS-PAGE, transferred on a nitrocellulose membrane (GE Healthcare) and blotted with an anti-ubiquitin antibody, or an anti-Syk antibody.

Production of recombinant Cbl-b²⁹⁻⁴⁸³ and C373A Cbl-b²⁹⁻⁴⁸³_{:} *Cbl-b²⁹⁻⁴⁸³* and C373A Cbl-b²⁹⁻⁴⁸³ coding sequences were cloned into the pGEX-2TK vector to obtain Gluthathione-S-transferase (GST) fusion proteins. Expression in Escherichia coli BL21 was induced with 0.3 mM isopropyl-D-thiogalactopyranoside at OD600 of 1.0 in Terrific Broth medium, and incubated overnight at 20°C. Cells were lysed in lysis buffer (20mM Tris-Hcl pH 7.5, 100mM NaCl, 1mM EDTA, 0.5% Triton X-100), sonicated briefly three times, and incubated overnight with Glutathione Sepharose 4B (GE Healthcare) at 4°C. Subsequently, Sepharose beads were washed 5 times with lysis buffer, and bound proteins were eluted with elution buffer (50mM Tris-Hcl, pH 8.0, and 20mM reduced glutathione). Eluates were tested for size and purity with SDS-PAGE and Western blotting.

Histopathology: For histopathology analyses, kidneys were fixed in 10% neutral buffered formalin, processed according to standard procedures, embedded in paraffin, and sectioned. 2 µm thick sections were stained with haematoxylin and eosin (H&E), periodic-acid-Schiff (PAS), or anti-Ly-6G (Biolegend; clone 1A8). Immunohistochemistry was performed with a primary antibody dilution of 1:100 with DAB enhancement after antigen retrieval (BOND Epitope Retrieval kit, Leica). 4 µm sections were stained with Gomori Methenamine Silver (GMS; Merck). Stained slides were scanned using a Pannoramic slide scanner (3D Histech) and evaluated for severity of inflammation and intra-lesional fungal burden based on the following criteria:

| **SCORE** | **RENAL INFLAMMATION (Based on H&E and PAS)** | **INTRALESIONAL FUNGAL BURDEN (Based on PAS and GMS)** |
|---|---|---|
| | Proportion of renal parenchyma and/or pelvis involved by tubulointerstitial nephritis and/or pyelonephritis | Extent of presence of intact fungal bodies (hyphae, pseudohyphae or spores) within and around foci of inflammation |
| 0 | None/Not significant | None/Not significant |
| 1 | Less than 10% | Scant presence in less than 10% of inflammatory foci |
| 2 | 10% to 25% | Mild to moderate presence in 10% to 25% of inflammatory foci |
| 3 | 25% to 50% | Moderate to significant presence in 25% to 50% of inflammatory foci |
| 4 | Greater than 50% | Significant presence in greater than 50% of inflammatory foci |

*Definiens Tissue Studio*TM software was applied to evaluate the extent of infiltration by Ly-6G⁺ cells in affected kidneys. Regions and cells of interest were manually classified in representative areas of slides to train the software algorithm to identify Ly-6G positive target objects and exclude non-target objects. Target objects were identified on the basis of morphology and positive immunohistochemical staining. The optimized algorithm was then applied to analyze all slides automatically. The validity of the analysis was confirmed by histopathologic verification of representative slides from both groups.

Peptide synthesis: Solid phase peptide synthesis (SPPS) was used for TKB binding peptide synthesis on an ABI 433a Peptide Synthesizer (Applied-Biosystems). Peptides were purified with an Ultimate 3000 RP-HPLC system (Thermo Scientific), and analyzed with a 4800 MALDI TOF/TOF analyzer (ABSciex). Lyophilized peptides were resolved in PBS and stored at -80°C.

Immunofluorescence: BM-CD were seeded on µ-slides (IBIDI) 24h before infection with *C. albicans.* After infection cells were fixed in 4% PFA on ice for 20min, permeabilized (0.2% Triton X-100 in PBS) for 10 min at room temperature, and blocked (3% BSA in PBS) for 1 hour at room temperature. Subsequently, cells were incubated with primary antibodies against Syk (1:100) or Shp-2 (1:100) in 3% BSA at overnight. Cells were then washed in PBS and incubated with an anti-rabbit-Alexa555 antibody (1:500) in 3% BSA at room temperature for 1h. After washing in PBS cells were counterstained with DAPI, and directly imaged with a Zeiss LSM780 Confocal microscope.

NanoLC-MS Analysis: The nano HPLC system used for this study was an UltiMate 3000 HPLC RSLC nano system (Thermo Scientific) coupled to a Q Exactive Plus mass spectrometer (Thermo Scientific), equipped with a Proxeon nanospray source (Thermo Scientific). Peptides were loaded onto a trap column (Thermo Fisher Scientific, Amsterdam, Netherlands, PepMap C18, 5 mm x 300 µm ID, 5 µm particles, 100 Å pore size) at a flow rate of 25 µL min⁻¹ using 0.1% TFA as mobile phase. After 10 min the trap column was switched in line with the analytical column (Thermo Fisher Scientific, Amsterdam, Netherlands, PepMap C18, 500 mm x 75 µm ID, 2 µm, 100 Å). Peptides were eluted using a flow rate of 230 nl min⁻¹, and a binary 2h gradient for 165 min. The gradient was started with 98% water/formic acid (99.9/0.1; v/v) and 2% water/acetonitrile/formic acid (19.92/80/0.08; v/v/v), and increased to 35% water/formic acid (99.9/0.1; v/v) and 65% water/acetonitrile/formic acid (19.92/80/0.08; v/v/v) over 120min, followed by a gradient to 10% water/formic acid (99.9/0.1; v/v) and 90% water/acetonitrile/formic acid (19.92/80/0.08; v/v/v) over 5min, kept at this ratio for 5 min and subsequently decreased over 2 min back to 98% water/formic acid (99.9/0.1; v/v) and 2% water/acetonitrile/formic acid (19.92/80/0.08; v/v/v) for equilibration at 30°C. The Q Exactive mass spectrometer was operated in data-dependent mode, using a full scan (m/z range 370-1650, nominal resolution of 70 000, target value 3E6) followed by MS/MS scans of the 12 most abundant ions. MS/MS spectra were acquired using normalized collision energy 27%, isolation width of 2 and the target value 1E5. Precursor ions selected for fragmentation (charge state 2 and higher) were put on a dynamic exclusion list for 10 sec. The underfill ratio was set to 20% resulting in an intensity threshold of 4E4. The peptide match feature and the exclude isotopes feature were enabled.

Mass spectrometry data analysis: For peptide identification the RAW-files were loaded into Proteome Discoverer (version 1.4.0.288, Thermo Scientific). All created MS/MS spectra were searched using the search engine node MSAmanda (Dorfer et al., 2014) against the flybase sequence database (22,256 sequences; 20,222,850 residues) and also against the Swissprot sequence database, using the taxonomy human (20,170 sequences, 11,318,213 residues). The following search parameters were used: Beta-methylthiolation on cysteine was set as a fixed modification, oxidation on methionine, acetylation on lysine, phosphorylation on serine, threonine and tyrosine, deamidation on asparagine and glutamine, and ubiquitinylation on lysine were set as variable modifications. Monoisotopic masses were searched within unrestricted protein masses for tryptic peptides. The peptide mass tolerance was set to ±5 ppm and the fragment mass tolerance to ± 0.03 Da. The maximal number of missed cleavages was set to 2. The result was filtered to 1% FDR using Percolator algorithm integrated in Proteome Discoverer. The localization of the sites of variable modifications within the peptides was performed with the tool ptmRS, integrated in Proteome Discoverer and based on phosphoRS (Taus et al., 2011).

Data Analysis and Statistics. All values in the paper are given as means ± standard deviation unless stated otherwise. All *in vitro* experiments were reproduced 3-5 independent times. *In vivo* experiments were reproduced 2-6 times. Figures and statistical analyses were generated using PraphPad Prism software (GraphPad Software). No mice were excluded from the analyses and histopathologic analyses using Definies Tissue studio software were performed blindly. Mice were allocated to experimental groups based upon their genotypes and randomized within their sex- and age matched groups. Since mice were inbred and sex- and age-matched, similar variance between experimental cohorts was assumed. An *a priori* sample size estimation was not performed. Group sizes were based upon the empirically assessed variability of the model systems or assays used, and groups contained as many mice as possible to minimize type I and type II errors. Data were analyzed using the unpaired two-tailed Student's *t*-test, or two way ANOVA, as indicated. For survival analyses, log rank tests were performed. P values < 0.05 were accepted as statistically significant.

The following antibodies were used in this study:

| **Antigen** | **Clone** | **Distributor** |
|---|---|---|
| Syk/SYK | D115Q | Cell Signaling Technologies |
| phospho Syk | C87C1 | Cell Signaling Technologies |
| Src | 36D10 | Cell Signaling Technologies |
| phospho Src | D7F2Q | Cell Signaling Technologies |
| Plc-γ2 | Q-20 | Santa Cruz Biotechnology |
| phospho-Plc-γ2 | Tyr1217 | Cell Signaling Technologies |
| NF-κB p65 | E498 | Cell Signaling Technologies |
| phospho-NF-κB p65 | 93H1 | Cell Signaling Technologies |
| Shp-2 | C-18 | Santa Cruz Biotechnology |
| phospho-Shp-2 | Tyr542 | Cell Signaling Technologies |
| β-Actin | 13 E 6 | Cell Signaling Technologies |
| Ubiquitin | P4D1 | Santa Cruz Biotechnology |
| Ly-6G FITC | 1A8 | BD Biosciences |
| CD8 Brilliant Violet 605 | 53-6.7 | Biolegend |
| TCRβ Brilliant violet 421 | H57-597 | BD Biosciences |
| NK-1.1 APC | PK136 | BD Biosciences |
| CD19 PE | 6D5 | BD Biosciences |
| CD11c FITC | HL3 | Biolegend |
| CD16/32 purified | 2.4G2 | BD Biosciences |
| Ly-6C PerCP-Cy5.5 | AL-21 | BD Biosciences |
| anti-rabbit Alexa555 | polyclonal | Invitrogen |

**The following PCR primers were used in this study:**

| **Target** | **fwd** | **rev** | **amplicon size** |
|---|---|---|---|
| Clec4n | CTGCCCAAATCACTGGAAGT (SEQ ID NO: 18) | GAAATTCTGCTCCGCTTCAG (SEQ ID NO: 19) | 142bp |
| Clec7a | GAACCACAAGCCCACAGAAT (SEQ ID NO: 20) | TAGGAAGGCAAGGCTGAGAA (SEQ ID NO: 21) | 95bp |
| Clec4d | TACGCTGGACGAGAGGAAGT (SEQ ID NO: 22) | ACAGGACAGCAGGTCCAAGT (SEQ ID NO: 23) | 112bp |
| Clec4e | CAAGTGCTCTCCTGGACGAT (SEQ ID NO: 24) | CTGTAAGTTCTGCCCGGAAA (SEQ ID NO: 25) | 114bp |
| TNF | CAAAATTCGAGTGACAAGCCTG (SEQ ID NO: 26) | GAGATCCATGCCGTTGGC (SEQ ID NO: 27) | 88bp |
| IL-6 | GAGGATACCACTCCCAACAGACC (SEQ ID NO: 28) | AAGTGCATCATCGTTGTTCATACA (SEQ ID NO: 29) | 140bp |
| IL-12 | GGAAGCACGGCAGCAGAATA (SEQ ID NO: 30) | AACTTGAGGGAGAAGTAGGAATGG (SEQ ID NO: 31) | 159bp |
| IL-23 | GCAGATTCCAAGCCTCAGTC (SEQ ID NO: 32) | TTCAACATATGCAGGTCCCA (SEQ ID NO: 33) | 86bp |
| IL-1β | CAACCAACAAGTGATATTCTCCA TG (SEQ ID NO: 34) | GATCCACACTCTCCAGCTGCA (SEQ ID NO: 35) | 120bp |

### Example 2 - Loss of Cbl-b or Cbl-b catalytic activity ameliorates fungal pathology

To test for a potential role of Cbl-b in anti-fungal immunity, *Cbl-b*^{*Δ*/*Δ*} and control *Cbl*-*b*^{+/+} littermates were systemically challenged with a lethal dose of *Candida albicans.* In addition, *Cbl-b*^{*c373A*/*c373A*} mice, carrying catalytically inactive Cbl-b (Paolino et al., 2011), were infected with *Candida albicans.* Loss of Cbl-b protein (Fig. 2a) or Cbl-b catalytic function markedly ameliorated fungal pathology as shown by decreased weight loss after infection (Fig. 1a) and by significantly delayed mortality (Fig. 1b) when compared to *Cbl-b*^{+/+} littermate animals. In line with these results, significantly reduced fungal burdens in kidneys, spleens, livers, and lungs of *Cbl-b*^{*Δ*/*Δ*} mice could also be observed, when compared to their *Cbl*-*b*^{+/+} littermate controls (Fig. 1c). Histopathologic analysis of affected kidneys 7 days after systemic infection confirmed these results; *Cbl-b*^{*Δ*/*Δ*} mice manifested reduced renal inflammation, as assessed by Periodic-acid-Schiff staining (Fig. 1d,e) and reduced numbers of *Candida albicans* yeasts and hyphae (Fig. 1f,g), as assessed with Gomori Methenamine Silver (GMS) staining. In line with a decreased fungal burden, a significant reduction in neutrophil infiltration was detected, as assessed by staining for the neutrophil specific marker Ly-6G on kidney sections of infected *Cbl-b*^{*Δ*/*Δ*} mice when compared to *Cbl-b^{+l+}* littermate controls (Fig. 1h, i). The protective effect of *Cbl-b* deficiency was not limited to infections with *Candida albicans,* since systemic infection with a lethal dose of a clinical isolate of *Candida dubliniensis* yielded similar results with respect to mortality rates and kinetics, when *Cbl-b*^{*Δ*/*Δ*} mice were compared to *Cbl-b*^{*+*/*+*} littermate controls (Fig. 2c). To assess, whether the protective effect of *Cbl-b* deficiency in severe fungal infection was the result of a broad deregulation of innate immune function, *Cbl-b* deficient and littermate control mice were infected with the gram-positive bacterium *Listeria monocytogenes.* Loss of Cbl-b did not affect the mortality of *Listeria monocytogenes* infected mice (Fig. 2d). The results show that loss of Cbl-b results in marked resistance to systemic *Candida albicans* and *Candida dubliniensis* infections.

To gain insight into the cellular basis of the observed protective effect, bone marrow chimeric animals were generated by lethally irradiating C57Bl/6 recipient mice and reconstituting them with bone marrow from syngeneic *Cbl-b*^{*Δ*/}*^{Δ}, Cbl-b*^{*C373A*/}*^{C373A},* or *Cbl-b*^{*+*/*+*} littermate mice. After reconstitution, the cohorts were infected with a lethal dose of *Candida albicans.* Loss of Cbl-b in the radiosensitive hematopoietic compartment mirrored full body *Cbl-b* deficiency (Fig. 3a,b). Due to the role of Cbl-b in adaptive immunity, it was next tested for a potential contribution of adaptive immune cells to the observed anti-fungal activity. To this end, *Cbl-b*^{*+*/*+*}/*Rag2*^{*Δ*/*Δ*} and *Cbi-b*^{*Δ*/*Δ*} /*Rag2*^{*Δ*/*Δ*} double deficient animals that lack an adaptive immune system were generated. Importantly, *Cbl-b* deficiency in the innate immune system, and in the absence of adaptive immunity, closely resembled the full body knock-out phenotype upon lethal infection with *Candida albicans* (Fig. 3c,d). Additionally, bone marrow chimeric animals were generated by lethal irradiation of *Rag2*^{*Δ*/*Δ*} recipients and reconstitution with *Cbl-b*^{*Δ*/*Δ*}/*Rag2*^{*Δ*/*Δ*} or *Cbl-b*^{*+*/*+*}/*Rag2*^{*Δ*/*Δ*} bone marrow grafts, to restrict potential effects of *Cbl-b* deficiency to the innate immune system. Again it could be observed that loss of Cbl-b confers a protective effect to systemic infection with *Candida albicans* (Fig. 3e,f), indicating that Cbl-b controls innate anti-fungal immunity.

To test for potential effects of *Cbl-b* deficiency on the development or maintenance of innate immune cells relevant for the anti-fungal defense, cellularity and cellular composition of the main innate immune lineages in the bone marrow and spleen of *Cbl-b*^{*Δ*/*Δ*} and *Cbl-b^{+l+}* littermates were assessed. Slightly elevated levels of both bone marrow and splenic Ly-6G⁺ neutrophils and splenic NK cells could be observed (Fig. 2e,f). To address whether this might affect the infiltration of monocytes or neutrophils, their recruitment in response to systemic *Candida albicans* challenge 24h after infection was quantified. At this early time point after infection, comparable, or even slightly decreased immune infiltration into kidneys, lungs, and livers of the *Cbl-b*^{*Δ*/*Δ*} mice could be observed (Fig. 4a,b,c). Recognition of *Candida albicans* cell wall β-glucan by the CLR family member Dectin-1 has been shown to be of crucial importance in the anti-fungal immune response (Brown et al., 2001). Comparable levels of Dectin-1 expression on peripheral blood monocytes and neutrophils of *Cbl-b*^{*Δ*/*Δ*} and control *Cbl-b*^{*+*/*+*} littermate mice were detected (Fig. 4d,e). Moreover, mRNA expression of Dectin-1 *(Clec7a),* as well as Dectin-2 (*Clec4n*), and Dectin-3 (*Clec4d*) as well as Mincle (*Clec4e*) were comparable in splenocytes and bone marrow cells of *Cbl-b*^{*Δ*/*Δ*} and *Cbl-b*^{*+*/*+*} animals, as assessed by qPCR (Fig. 4f, g). Importantly, although *Cbl-b*^{*Δ*/*Δ*} and *Cbl-b*^{*+*/*+*} animals exhibited comparable early cellular recruitment patterns in response to systemic *Candida albicans* challenge, fungal burdens in affected organs were already significantly decreased 24 hours after infection (Fig. 4h), supporting the notion that loss of Cbl-b results in an increased fungicidal activity of innate immune cells.

### Example 3 - Loss of Cbl-b confers resistance to both systemic and cutaneous fungal infections

To test whether the protective effect of *Cbl-b* deficiency could be extended to other routes of infection, a model of cutaneous *Candida albicans* infection was used (Igyarto et al., 2011). In this model an increased resistance of *Cbl-b*^{*Δ*/*Δ*} mice to skin colonization by *Candida albicans* was again observed, when compared to littermate controls, as evidenced by a significantly decreased fungal burden (Fig. 3g). Since immune reactions against cutaneous infection with *Candida albicans* are characterized by a crosstalk between adaptive and innate immune cells (Igyarto et al., 2011; Kashem et al., 2015), *Candida albicans* skin infections were performed on a *Rag2* deficient background. *Cbl-b*^{*+*/*+*}/*Rag2*^{*Δ*/*Δ*} deficient animals harbored a slightly increased fungal burden when compared to *Cbl-b*^{*+*/*+*}/*Rag2*^{*+*/*+*} mice, although this did not reach statistical significance. Importantly, it was again observed that deletion of *Cbl-b* in the absence of an adaptive immune system had a protective effect with regard to fungal colonization, as evidenced by the decreased fungal load in the skin of *Cbl-b*^{*Δ*/*Δ*}/*Rag2*^{*Δ*/*Δ*} mice, when compared to their *Cbl-b*^{*+*/*+*}/*Rag2*^{*Δ*/*Δ*} littermate controls (Fig. 3h). Thus, loss of Cbl-b in innate immune cells confers resistance to systemic as well as cutaneous *Candida albicans* infections.

### Example 4 - Cbl-b controls anti-fungal activities of dendritic cells and macrophages

Fungicidal activity within the innate immune system is mainly attributed to neutrophils, macrophages and DC (Drummond et al., 2015). To directly investigate the effect of *Cbl-b* deficiency on the anti-fungal activity of these cell types, *in vitro* killing assays were performed by co-culturing bone marrow neutrophils from uninfected mice, bone marrow-derived macrophages (BM-M), bone marrow-derived dendritic cells (BM-DC), bone marrow monocytes, and splenic DC, isolated from the bone marrow or the spleen, respectively, of uninfected mice, with *Candida albicans.* No significant difference in fungicidal activity could be observed, when comparing *Cbl-b*^{*Δ*/}*^{Δ}, Cbl-b*^{*C373A*/*C373A*}, and *Cbl-b*^{*+*/*+*} bone marrow neutrophils (Fig. 5a). However, BM-M, and BM-DC, bone marrow monocytes and splenic DC, both isolated from uninfected mice, showed an increased potency to kill *Candida albicans* in the absence of Cbl-b or Cbl-b ligase function (Fig. 5b,c,d,e). Of note, rates of phagocytosis were comparable between BM-M or BM-DC of all three genotypes (Fig. 6a,b) . Compatible with a more prominent role for Cbl-b in anti-fungal responses by DC and macrophages, higher levels of Cbl-b protein in BM-DC and BM-M were observed, when compared to bone marrow neutrophils, both in lysates from *Candida albicans* stimulated as well as in uninfected cells (Fig. 6c). Thus, Cbl-b controls the fungicidal activity of both BM-M and BM-DC as well as isolated bone marrow derived monocytes and splenic DC.

In line with previous observations, pharmacologic inhibition of phagocytosis using Dynasore abrogated BM-DC mediated killing. Likewise, antibody mediated blocking of Dectin-1 significantly reduced *Candida albicans* killing and reduced the increased fungal killing in both *Cbl-b*^{*Δ*/*Δ*} and *Cbl-b*^{*C373A*/*C373A*} BM-DC to levels observed in *Cbl*-*b*^{+/+} cells (Fig. 5f). In addition, pharmacologic inhibition of Syk with R406 almost completely abrogated *Candida albicans* killing (Fig. 5g). It was next measured whether a lack of Cbl-b or defective Cbl-b ligase function affects the ability of BM-M, BM-DC, bone marrow-derived monocytes, and splenic DC to produce ROS. *Cbl-b*^{*Δ*/*Δ*} or *Cbl-b*^{*C373A*/*C373A*} bone marrow neutrophils did not significantly differ from *Cbl-b*^{*+*/*+*} bone marrow neutrophils with respect to both the kinetics and amplitude of ROS production in response to *Candida albicans* (Fig. 6d). However, the increased fungicidal activity of *Cbl-b*^{*Δ*/*Δ*} or *Cbl-b*^{*C373A*/*C373A*} BM-M, BM-DC, bone marrow monocytes and splenic DC correlated with an increased *Candida albicans-*induced ROS production as compared to Cbl-b sufficient cells (Fig. 6e-h). To test whether this deregulated cellular response to fungal challenge is specific to stimulation with *Candida albicans,* BM-DC were stimulated with clinical isolates of *Candida glabrata, Candida krusei,* and *Candida dubliniensis.* It was again observed, that loss of Cbl-b significantly enhanced the production of ROS in response to stimulation with these distinct fungi (Fig. 6i-k). Thus, loss of Cbl-b enhances the immune response to several clinically relevant fungal pathogens.

*Candida albicans* mediated activation of BM-DC also triggers Syk dependent activation of the inflammasome, that enables cleavage of pro-interleukin-1β (IL-1β) by Caspase-8 (Gringhuis et al., 2012; Zwolanek et al., 2014). Significantly increased activation of Caspase-8 (Fig. 7a) and enhanced release of mature IL-1β in *Candida albicans* stimulated *Cbl-b*^{*Δ*/*Δ*} or *Cbl-b*^{*C373A*/*C373A*} BM-DC were indeed observed when compared to control *Cbl-b*^{*+*/*+*} BM-DC, which could be reduced by a Dectin-1 blocking antibody (Fig. 7b). Pharmacologic inhibition of Syk also almost completely abrogated IL-1β secretion from BM-DC of all three genotypes (Fig. 7c). Similarly increased production and release of mature IL-1β from *Candida albicans*-stimulated *Cbl-b*^{*Δ*/*Δ*} or *Cbl-b*^{*C373A*/*C373A*} BM-M were found, when compared to Cbl-b^{+/+} BM-M (Fig. 7d).

To monitor the transcriptional response to *Candida albicans* infection, mRNA levels of pro-inflammatory cytokines were assessed. Significantly increased transcript levels of IL-1β, IL-6, TNF, IL-23, and IL-12 in *Cbl-b*^{*Δ*/*Δ*} and *Cbl-b*^{*C373A*/*C373A*} BM-DC when compared to *Cbl*-*b*^{+/+} control cells were observed (Fig. 7e). This increase in cytokine mRNA expression correlated with significantly increased secretion of the pro-inflammatory cytokines IL-6 and TNF by *Cbl-b*^{*Δ*/*Δ*} and *Cbl-b*^{*C373A*/*C373A*} BM-DC when compared to *Cbl*-*b*^{+/+} BM-DC (Fig. 7f,g) . Blocking Dectin-1 reduced the release of IL-6 and TNF for all three genotypes and, importantly, also reduced the increased cytokine release in the *Cbl-b* deficient BM-DC (Fig. 7f,g). Again, pharmacologic inhibition of Syk almost completely abrogated the release of IL-6 and TNF by *Candida albicans* stimulated BM-DC of all three genotypes (Fig. 7h,i). *Candida albicans* stimulation also led to a substantially increased release of IL-23 and IL-12 by BM-DC (Fig. 7j,k) and IL-6 and TNF by BM-M (Fig. 7l,m), when *Cbl-b*^{*Δ*/*Δ*} and *Cbl-b*^{*C373A*/*C373A*} cells were compared to *Cbl-b*^{*+*/*+*} control cells. Additionally the secretion of the anti-inflammatory mediator IL-1 receptor antagonist (IL-1Ra) by *Candida albicans* stimulated BM-DC was tested. A significant increase in secretion of IL-1Ra was observed when *Cbl-b*^{*Δ*/*Δ*} and *Cbl-b^{+l+}* BM-DC were compared (Fig. 7n).

To further test for the contribution of Dectin-1, cytokine secretion by BM-DC of all three genotypes in response to increasing doses of zymosan, a *Candida albicans* cell wall homogenate, or curdlan, a purified β-glucan was compared. Stimulation with zymosan and the Dectin-1 agonist curdlan resulted in an increased release of IL-1β, IL-6, and TNF by *Cbl-b*^{*Δ*/*Δ*} and *Cbl-b*^{*C373A*/*C373A*} BM-DC (Fig. 8a-f). Since loss of Cbl-b did not affect the mortality rates in lethal *Listeria monocytogenes* infections (see Fig. 2d), a potential role of Cbl-b in the response of BM-DC to stimulation with the TLR4 agonist Lipopolysaccharide (LPS) or the TLR9 agonist CpG was tested, both important pathogen associated molecular pattern receptors involved in *Listeria monocytogenes* infections. Importantly, no significant differences in the secretion of the pro-inflammatory cytokines IL-6 and TNF in response to stimulation with a high or low dose of LPS or CpG were observed, when *Cbl-b*^{*Δ*/*Δ*} and *Cbl-b*^{*+*/*+*} BM-DC were compared (Fig. 8g-j). Thus, Cbl-b is a key regulator of Dectin-1/Syk-mediated responses of BM-DC and BM-M to *Candida albicans* infections.

The data so far showed that *Cbl-b* deficiency or a lack of *Cbl-b* activity leads to a severe de-regulation of the anti-fungal immune response *in vitro.* To address, whether the observed differences could be recapitulated in immune cells recruited to inflammatory sites, *Cbl-b*^{*Δ*/*Δ*} and *Cbl*-*b*^{+/+} mice were challenged by intraperitoneal infection with *Candida albicans* and their anti-fungal activity was assessed 24h later. Loss of *Cbl-b* led to an enhanced ROS response by total infiltrating immune cells into the peritoneal cavity. Increased ROS production was already observed in *Cbl-b*^{*Δ*/}*^{Δ},* when compared to *Cbl*-*b*^{+/+}, infiltrating cells without further re-stimulation with *Candida albicans* (Figure 5h,i). Likewise, the fungicidal activity of *Cbl-b*^{*Δ*/*Δ*} immune infiltrates was increased, when compared to immune cell infiltrates from *Cbl-b*^{*+*/*+*} littermate control mice (Figure 5j). Intraperitoneal infection with *Candida albicans* leads to the recruitment of both inflammatory monocytes/macrophages and neutrophils. To address the contribution of either cell type to the observed differences, neutrophils and monocytes/macrophages were FACS purified from total infiltrating immune cells and tested for their ability to produce ROS after re-stimulation with *Candida albicans.* An increase in ROS production by *Cbl-b*^{*Δ*/*Δ*} monocytes/macrophages was observed, but not neutrophils, recruited to the peritoneal cavity when compared to *Cbl-b*^{*+*/*+*} cells (Fig. 5k,l). Additionally, intraperitoneal lavages were tested for the inflammatory cytokines IL-6 and TNF, as well as IL-1Ra. As seen in the *in vitro* experiments, an increase in both IL-6 and TNF was observed, as well as IL-1Ra, when peritoneal lavages from *Cbl-b*^{*Δ*/*Δ*} and *Cbl-b*^{*+*/*+*} mice were compared (Fig. 8k).

The data indicated that the protective effect of *Cbl-b* deficiency to fungal challenge was mediated by tissue resident or recruited phagocytic immune cells, but not neutrophils. To test for neutrophil intrinsic effects phagocytic immune cells were depleted *in vivo* using clodronate liposomes. Clodronate liposomes have been reported to deplete macrophages, subpopulations of monocytes, and subsets of DC, while leaving neutrophils unaffected (Buiting et al., 1994; Buiting et al., 1996; Qian et al., 1994; Sunderkotter et al., 2004; van Rooijen et al., 1992). Phagocytes were depleted 24h before, and 24h after systemic infection with *Candida albicans* and both weight loss and survival were monitored over time. Depletion of phagocytes led to a significant increase in weight loss in both *Cbl*-*b*^{+/+} and *Cbl-b*^{*Δ*/*Δ*} mice compared to the control PBS liposome treated cohorts (Qian et al., 1994) (Figure 5m). Importantly, there was no significant difference between *Cbl-b*^{*Δ*/*Δ*} and *Cbl-b*^{*+*/*+*} animals treated with clodronate liposomes (Figure 5m). Likewise, while *Cbl-b* deficiency led to a prolonged survival when PBS liposome treated *Cbl-b*^{*Δ*/*Δ*} and *Cbl*-*b*^{+/+} cohorts were compared, depletion of phagocytes by clodronate liposomes completely abolished any survival benefit due to the loss of *Cbl-b* (Figure 5n). These data show that *Cbl-b* deficiency in phagocytic cells is critical for the observed beneficial effect to systemic *Candida albicans* infections.

### Example 5 - Loss of Cbl-b enhances CLR-mediated signaling in response to Candida albicans

To assess whether loss of Cbl-b or Cbl-b ligase function altered the magnitude of the signaling response mounted by BM-DC, tyrosine phosphorylation was analyzed 30, 60, and 90 minutes after stimulation with *Candida albicans.* A strong increase in tyrosine phosphorylation in response to *Candida albicans* stimulation was observed in lysates prepared from *Cbl-b*^{*Δ*/*Δ*} and *Cbl-b*^{*C373A*/*C373A*} BM-DC, when compared to control Cbl-b^{+/+} BM-DC for all stimulation time points tested (Fig. 9a). Canonical signaling by CLRs involves proximal activation of Src kinase and Shp-2 that subsequently results in the activation of Syk (Deng et al., 2015). Comparable Src tyrosine phosphorylation was observed after stimulation of BM-DC with *Candida albicans,* but increased levels of phosphorylated Shp-2 and Syk (Fig. 9b). Due to the increased anti-fungal effects caused by Syk inhibition as compared to Dectin-1 blockade, the role of Cbl-b deletion in Dectin-1, Dectin-2/3, and Mincle signaling was further tested. Although these surface receptors were expressed at comparable levels in *Cbl-b*^{*Δ*/*Δ*} and *Cbl-b*^{*+*/*+*} BM-DC (Fig. 10a), an increase in Syk and Shp-2 phosphorylation in response to heat killed *Candida albicans* (HK; to activate Dectin-1), *Candida albicans* hyphae (Hyph; to activate Dectin-2/3), as well as Trehalose-6,6-dimycolate (TDM; to activate Mincle) in the absence of Cbl-b (Fig. 9c) was observed. Additionally, an increase in phosphorylation of the distal CLR signaling molecules PLCγ2 and p65 NF-κB in *Candida albicans*-stimulated *Cbl-b*^{*Δ*/*Δ*} and *Cbl-b*^{*C373A*/*C373A*} BM-DC was observed (Fig. 9d). In agreement with the immunoblots, *Cbl-b*^{*Δ*/*Δ*} BM-DC showed increased levels of both phosphorylated Shp-2 (Fig. 9e) and phosphorylated Syk (Fig. 9f) following stimulation with *Candida albicans,* as analyzed by confocal microscopy. These data identify a central function for Cbl-b in regulating proximal CLR signaling in response to *Candida albicans* infection.

### Example 6 - Cbl-b targets Syk and Dectin-1, and -2 for ubiquitination

To test whether the E3 ligase Cbl-b could poly-ubiquitinate molecules critical for proximal CLR signaling, ubiquitination assays with recombinant proteins were performed. No poly-ubiquitination of recombinant Shp-2 was detected in these assays, but observed robust poly-ubiquitination of recombinant active Syk was observed (Fig. 11a). The chain linkage type formed by Cbl-b was analyzed by mass spectrometry and unique peptides (FAGKQLED) (SEQ ID NO: 36) derived specifically from K48-linked ubiquitin chains were identified (Fig. 10b). Cbl-b substrate specificity is mainly mediated by the amino-terminal tyrosine kinase binding (TKB) domain. In agreement with this notion, a truncated Cbl-b protein (Cbl-b²⁹⁻⁴⁸³), lacking the carboxy-terminal ubiquitin associated (UBA) domain, but containing the TKB and RING finger domains, still exhibited poly-ubiquitination activity towards Syk (Fig. 11a). As expected, the C373A mutant of Cbl-b²⁹⁻⁴⁸³ did not show Syk poly-ubiquitination activity (Fig. 11a). Importantly, non-phosphorylated recombinant Syk showed strongly diminished poly-ubiquitination by Cbl-b²⁹⁻⁴⁸³ (Fig. 11b), consistent with Cbl-b targeting activated Syk. To test for interactions between Syk and Cbl-b, co-immunoprecipitation experiments were performed. Increased co-immunoprecipitation of Syk with Cbl-b after stimulation of BM-DC with *Candida albicans* could be observed (Fig. 11c). Upon treatment with the proteasome blocker MG132, an increase in phosphorylated Syk, but not total Syk, was observed in lysates from *Cbl-b^{+l+}* BM-DC stimulated with *Candida albicans.* Importantly, levels of phosphorylated Syk did not change upon MG132 treatment of *Cbl-b*^{*Δ*/*Δ*} BM-DC stimulated with *Candida albicans* (Fig. 11d), indicating that Cbl-b mediates its effects via proteasomal targeting. These results show that Cbl-b can ubiquitinate active Syk.

To test for potential additional effects of *Cbl-b* deletion on proximal signaling initiated by CLR stimulation, the question was raised whether the CLR family members Dectin-1, and -2 might also serve as targets for Cbl-b mediated degradation. Thus, BM-M were stimulated with *Candida albicans* or *Candida albicans* hyphae, and tested for Dectin-1, and -2 protein levels, respectively, at various time points after stimulation. While Dectin-1, and -2 levels decreased over time in control cells, their expression levels remained constant in the absence of Cbl-b (Fig. 10c,d). To test whether Dectin-1, and -2 are ubiquitinated in response to fungal stimulation, and if so, whether ubiquitination is dependent on Cbl-b, *Cbl-b*^{*Δ*/*Δ*} and *Cbl-b*^{*+*/*+*} cells were stimulated, Dectin-1, or -2 were immunoprecipitated and ubiquitin was blotted. Ubiquitination of Dectin-1 and Dectin-2 in response to stimulation with *Candida albicans* or *Candida albicans* hyphae, respectively, was observed. This ubiquitination was reduced in the absence of Cbl-b (Fig. 10e,f). These data show that Cbl-b regulates signaling of CLRs by both regulating the protein levels of Dectin-1, and -2, as well as modulating the proximal signaling molecule Syk.

### Example 7 - Inhibition of Cbl-b enhances anti-fungal immunity in vitro and in vivo

Based on the genetic and biochemical data it was reasoned that blocking Cbl-b-TKB mediated substrate recognition might deregulate the activation of Syk downstream of CLR stimulation. To test this hypothesis, a fusion peptide consisting of a cell penetrating peptide derived from the *Drosophila* Antennapedia homeodomain was designed (May et al., 2000) and a 16aa long phosphorylated peptide derived from Syk that encompasses Tyrosine 317 (Y317), reported to be required for Cbl-family-ligase mediated targeting of Syk (Sohn et al., 2003; Yankee et al., 1999; Zou et al., 2009), and the surrounding flanking regions (Fig. 11e). Attachment of the Antennapedia peptide has been reported to allow for the efficient delivery of attached peptides into the cytosol (Jones et al., 2005). Pre-incubation of recombinant Cbl-b with this Cbl-b TKB-binding peptide completely abrogated *in vitro* ubiquitination of active Syk (Fig. 11f). Since activation of ROS production in BM-DC in response to fungal stimulation is Syk dependent, ROS production in the presence of the novel Cbl-b-TKB binding peptide was monitored. Intriguingly, peptide interference massively increased ROS production by BM-DC in response to *Candida albicans* stimulation (Fig. 11g). The observed effect even surpassed the increase in ROS production in *Cbl-b*^{*Δ*/*Δ*} cells. Since the peptide interference strategy potentially inhibits TKB mediated targeting of substrate proteins by all Cbl family ligases (c-Cbl, Cbl-b, and Cbl-3), it appears that Cbl family ligases could act in an at least partly redundant manner. In agreement with such a scenario, a significantly increased sensitivity to peptide interference in BM-DC lacking Cbl-b when compared to Cbl-b sufficient cells was observed (Fig. 10g). Most importantly, the Cbl-b TBK binding peptide also increased the fungicidal activity of BM-DC upon *Candida albicans* infection *in vitro* (Fig. 11h). Of note, addition of the Antennapedia peptide alone, or a phosphorylated scrambled peptide, did not affect ROS production, or the fungicidal activity of BM-DC.

Due to the observed *in vitro* efficacy of the inhibitory peptide it was investigated whether inhibition of Cbl family ligases might represent a novel strategy to increase immune reactivity and function towards fungal sepsis *in vivo.* Thus, *Cbl-b*^{*Δ*/*Δ*} and *Cbl-b*^{*+*/*+*} littermate control mice were infected with a lethal dose of *Candida albicans* and treated two times with a high dose of the inhibitory Cbl-b TKB-binding peptide (100µg, i.p., day 0 and day 2), or left untreated. Strikingly, treating mice only twice had a significant protective effect on wild type *Cbl-b^{+l+}* mice, as evidenced by reduced weight loss and delayed mortality, when compared to the untreated control cohort (Fig. 12a,b). Additionally, peptide treatment led to a decrease in blood urea nitrogen levels, indicative of reduced kidney damage (Fig. 12c). Peptide treatment did not confer any protective effect on *Cbl-b*^{*Δ*/*Δ*} mice (Fig. 10h). Reducing the concentration of the TKB binding inhibitor by 5-fold (20µg per injection) yielded comparable results (Fig. 12d,e; Fig. 10i). In line with the decreased morbidity observed upon low dose peptide treatment, a significantly reduced fungal load in the kidneys of peptide treated mice was detected (Fig. 12f). Treating mice with the Antennapedia peptide or a scrambled phosphorylated control peptide did not affect morbidity or mortality, when compared to the untreated control cohort. Due to the protective function of peptide treatment in *Cbl-b^{+l+}* mice, the therapeutic potential of the TKB-binding peptide in already established fungal sepsis was finally tested. Thus, mice were treated on day 2 and day 3 (100µg per i.p. injection) after infection with a lethal dose of *Candida albicans.* Strikingly, this treatment regimen completely blocked further weight loss, significantly reduced kidney fungal load 2 days after the second peptide treatment, and even protected mice from mortality (Fig. 12g,h,i). Histopathologic analyses confirmed these findings, showing significantly reduced renal inflammation (Fig. 12j,k), fungal burden (Fig. 10j), and neutrophil infiltration (Fig. 10k). Thus, the Cbl-b-TBK binding peptide enhances anti-fungal immunity and can protect from lethal *Candida albicans* sepsis.

### References

Armstrong-James et al. Trends in microbiology 22, 120-127 (2014).
Bachmaier et al. Nature 403, 211-216 (2000).
Bechara et al. FEBS letters 587, 1693-1702 (2013).
Bourgeois et al. Methods in molecular biology 470, 125-139 (2009) .
Boxio et al. Journal of leukocyte biology 75, 604-611 (2004).
Brown et al. Nature 413, 36-37 (2001).
Brown et al. Science translational medicine 4.165 (2012):165rv113.
Brown et al. Trends in microbiology 22, 107-109 (2014).
Buiting et al. Journal of drug targeting 2, 357-362 (1994).
Buiting et al. Journal of immunological methods 192, 55-62 (1996).
Chiang et al. Nature 403, 216-220 (2000).
Chiba et al. eLIFE 1-20 (2014).
Deng et al. Nature immunology 16, 642-652 (2015).
Denning Clin Infect Dis 26(4), 781-803 (1998).
Dorfer et al. Journal of proteome research 13, 3679-3684 (2014).
Drummond et al. Cold Spring Harbor perspectives in medicine 5(2015).
Enoch et al. Journal of Medical Microbiology 55, 809-818 (2006).
Gringhuis et al. Nature immunology 13, 246-254 (2012).
Gross et al. Nature 459, 433-436 (2009).
Gustin et al. Journal of immunology 177, 5980-5989 (2006).
Han et al. Nature immunology 11, 734-742 (2010).
Igyarto et al. Immunity 35, 260-272 (2011).
Jain et al. J Pharm Bioallied Sci. 2(4), 314-320 (2010).
Jones et al. British journal of pharmacology 145, 1093-1102 (2005).
Kashem et al. Immunity 42, 356-366 (2015).
Kawai et al. Nature Immunology 11, 373-384 (2010).
Kawai et al. Immunity 34(5), 637-650 (2011).
Kitaura et al. Immunity 26, 567-578 (2007).
Kullberg et al. The New England journal of medicine 373, 1445-1456 (2015).
Lanternier et al. Current opinion in pediatrics 25, 736-747 (2013).
LeibundGut-Landmann et al. Nature immunology 8, 630-638 (2007).
Lim et al. Nature Communications 6, 1-13 (2015).
Lindsay Curr. Opin. Pharmacol. 2, 587-594 (2002).
Loeser et al. Cell cycle 6, 2478-2485 (2007).
Lortholary et al. Clinical microbiology reviews 10, 477-504 (1997).
Mancini et al. Journal of Biological Chemistry 277, 14635-14640 (2002).
May et al. Science 289, 1550-1554 (2000).
Mocsai et al. Nature reviews. Immunology 10, 387-402 (2010).
Nakao et al. Molecular and cellular biology 29, 4798-4811 (2009).
Ohno et al. Archives of Biochemistry and Biophysics 594, 1-7 (2016).
Paolino et al. Journal of immunology 186, 2138-2147 (2011).
Paolino et al. Nature 507, 508-512 (2014).
Park et al. Aids 23, 525-530 (2009).
Peschard et al. Mol. Cell 8, 995-1004 (2001).
Peschard et al. Journal of Biological Chemistry 279, 29565-71 (2004).
Qian et al. Journal of immunology 152, 5000-5008 (1994).
Qu et al. Blood 103, 1779-1786 (2004).
Ravikumar et al. Frontiers in microbiology 6, 1322 (2015).
Ruland et al. Annals of the New York Academy of Sciences 1143, 35-44 (2008).
Sohn et al. The Journal of experimental medicine 197, 1511-1524 (2003).
Sunderkotter et al. Journal of immunology 172, 4410-4417 (2004).
Taus et al. Journal of proteome research 10, 5354-5362 (2011).
Tsygankov et al. Oncogene 20, 6382- 6402 (2001).
van Rooijen et al. Research in immunology 143, 215-219 (1992).
Wallner et al. PloS one 8, e65178 (2013).
Wirnsberger et al. Nature genetics 46, 1028-1033 (2014).
Yankee et al. Journal of immunology 163, 5827-5835 (1999).
Zou et al. The Journal of biological chemistry 284, 18833-18839 (2009).
Zwolanek et al. PLoS pathogens 10, e1004525 (2014). All documents cited herein are included herein by reference in their entirety.

## Claims

1. A fusion peptide comprising a cell delivery portion and a casitas b-lineage lymphoma-b (Cbl-b) binding peptide, wherein the Cbl-b binding peptide binds to the tyrosine kinase binding (TKB) domain of Cbl-b, preferably wherein the cell delivery portion is a cell penetrating peptide, more preferably wherein the Cbl-b binding peptide is a peptide fragment of spleen tyrosine kinase (Syk).

2. The fusion peptide according to claim 1, wherein the Cbl-b binding peptide has the sequence motif N/DXpY, preferably N/DX₁PYX₂P, wherein X, X₁ and X₂ are selected from any amino acid, N/D is either asparagine (N) or aspartic acid (D), and pY is phosphotyrosine.

3. The fusion peptide according to claim 1 or 2, wherein the Cbl-b binding peptide comprises the following consensus sequence:
SFNPYEPX₁X₂X₃,
wherein X₁-X₃ are selected independently from any amino acid, preferably wherein the Cbl-b binding peptide is a peptide fragment of Syk, more preferably wherein the Cbl-b binding peptide comprises the sequence SFNPYEPTGG, SFNPYEPTGG, SFNPYEPEVA or SFNPYEPELA.

4. The fusion peptide according to claim 3, wherein the tyrosine in said sequence is phosphorylated.

5. The peptide according to any one of claims 1 to 4, wherein the Cbl-b binding peptide is of Syk and wherein the peptide is a regulator of innate immunity, preferably a regulator of innate anti-parasitic, anti-fungal or anti-tumor immunity.

6. A pharmaceutical composition, comprising the fusion peptide or the Cbl-b binding peptide as set forth in any one of claims 1 to 5, and a pharmaceutically acceptable carrier promoting cellular uptake of said peptide, preferably wherein the pharmaceutically acceptable carrier is selected from vesicles, micelles, liposomes and microsomes, more preferably wherein the pharmaceutical composition is prepared for parenteral administration, especially intravenous, intraarterial, intramuscular, intrathecal, subcutaneous or intraperitoneal administration or for topical administration, preferably further comprising a skin penetration enhancer.

7. The peptide or pharmaceutical composition according to any one of claims 1 to 6, for use in therapy, wherein the peptide or composition is administered to a patient, preferably wherein the peptide is a Cbl-b binding peptide of Syk.

8. The peptide or pharmaceutical composition for use according to claim 7 for use in the prevention or treatment of tumors, cancer, viral infections, bacterial infections, fungal infections, parasitic infections, especially intracellular parasites, or drug-induced immunosuppression in a patient.

9. A Cbl-b inhibitor for use in therapy in a patient as a vaccine adjuvant, preferably wherein the Cbl-b inhibitor is a Cbl-b binding peptide, more preferably a Cbl-b binding peptide of Syk.

10. A Cbl-b inhibitor for use in the prevention or treatment of a fungal infection in a patient, wherein the fungal infection is a yeast infection, preferably a candidiasis or a cryptococcosis.

11. A Cbl-b inhibitor for use in the prevention or treatment of a fungal infection in a patient, wherein the Cbl-b inhibitor comprising a Cbl-b binding peptide of Syk.

12. The Cbl-b inhibitor or peptide or pharmaceutical composition for use according to any one of claims 7 to 11, wherein the fungal infection is a yeast infection, preferably a candidiasis, especially a *Candida albicans, Candida glabrata, Candida krusei* or a *Candida dubliniensis* infection, a dermatophyte or an aspergillus infection.

13. A vaccine, comprising as an adjuvant a Cbl-b inhibitor, preferably wherein the Cbl-b inhibitor comprises a Cbl-b binding peptide of Syk, and at least one antigen, preferably the Cbl-b inhibitor being defined as in any one of claims 1 to 5 and 7 to 12.

14. A kit comprising the peptide according to claims 1 to 13 and a pharmaceutically acceptable excipient, preferably suitable for intracellular administration in the patient.

15. An *in vitro* or *in vivo* method for stimulating or activating an immune cell, comprising contacting or treating the cell with a fusion peptide or a Cbl-b binding peptide as set forth in any one of claims 1 to 5, preferably wherein cytokines are produced, more preferably wherein the cytokines are selected from IL-6, IL-1β, IL-23 and TNF.


SEQUENCE LISTING

<110> IMBA - Institut für molekulare Biotechnologie GMBH

<120> Cbl-b inhibitor comprising a Cbl-b binding peptide for use as a
medicament

<130> r68932

<160> 36

<170> BiSSAP 1.3

<210> 1
<211> 10
<212> PRT
<213> Artificial Sequence

<220>
<223> consensus sequence

<220>
<221> VARIANT
<222> 8..10
<223> X is any amino acid

<400> 1
Ser Phe Asn Pro Tyr Glu Pro Xaa Xaa Xaa
1               5                   10

<210> 2
<211> 10
<212> PRT
<213> Mus musculus

<220>
<223> also in Rattus norvegicus

<400> 2
Ser Phe Asn Pro Tyr Glu Pro Thr Gly Gly
1               5                   10

<210> 3
<211> 10
<212> PRT
<213> Macaca mulatta

<400> 3
Ser Phe Asn Pro Tyr Glu Pro Glu Val Ala
1               5                   10

<210> 4
<211> 10
<212> PRT
<213> Homo sapiens

<400> 4
Ser Phe Asn Pro Tyr Glu Pro Glu Leu Ala
1               5                   10

<210> 5
<211> 16
<212> PRT

<213> Artificial Sequence

<220>
<223> consensus sequence

<220>
<221> VARIANT
<222> 11,12,13,14,16
<223> X is any amino acid

<400> 5
Ser Thr Val Ser Phe Asn Pro Tyr Glu Pro Xaa Xaa Xaa Xaa Trp Xaa
1               5                   10                  15

<210> 6
<211> 16
<212> PRT
<213> Mus musculus

<400> 6
Ser Thr Val Ser Phe Asn Pro Tyr Glu Pro Thr Gly Gly Pro Trp Gly
1               5                   10                  15

<210> 7
<211> 16
<212> PRT
<213> Rattus norvegicus

<400> 7
Ser Thr Val Ser Phe Asn Pro Tyr Glu Pro Thr Gly Gly Ala Trp Gly
1               5                   10                  15

<210> 8
<211> 16
<212> PRT
<213> Macaca mulatta

<400> 8
Ser Thr Val Ser Phe Asn Pro Tyr Glu Pro Glu Val Ala Pro Trp Ala
1               5                   10                  15

<210> 9
<211> 16
<212> PRT
<213> Homo sapiens

<400> 9
Ser Thr Val Ser Phe Asn Pro Tyr Glu Pro Glu Leu Ala Pro Trp Ala
1               5                   10                  15

<210> 10
<211> 17
<212> PRT

<213> Drosophila melanogaster

<400> 10
Asp Arg Gln Ile Lys Ile Trp Phe Gln Asn Arg Arg Met Lys Trp Lys
1               5                   10                  15
Lys


<210> 11
<211> 13
<212> PRT
<213> Human immunodeficiency virus 1

<400> 11
Gly Arg Lys Lys Arg Arg Gln Arg Arg Arg Pro Pro Gln
1               5                   10

<210> 12
<211> 27
<212> PRT
<213> Mammalia

<400> 12
Gly Trp Thr Leu Asn Ser Ala Gly Tyr Leu Leu Gly Lys Ile Asn Leu
1               5                   10                  15
Lys Ala Leu Ala Ala Leu Ala Lys Lys Ile Leu
            20                  25

<210> 13
<211> 11
<212> PRT
<213> Artificial Sequence

<220>
<221> REPEAT
<222> 1-11
<223> 1 to 5 times repeat

<220>
<223> consensus sequence

<220>
<221> VARIANT
<222> 10
<223> X is K or R

<400> 13
Lys Lys Asp Lys Lys Asp Glu Arg Arg Xaa Lys
1               5                   10

<210> 14
<211> 12
<212> PRT
<213> Artificial Sequence

<220>
<221> REPEAT
<222> 1..11
<223> 1 to 5 times repeat

<220>
<223> consensus sequence

<220>
<221> VARIANT
<222> 5
<223> X is V, I or T

<400> 14
Lys Ile Lys Lys Xaa Asx Lys Lys Lys Gly Arg Lys
1               5                   10

<210> 15
<211> 5
<212> PRT
<213> Artificial Sequence

<220>
<223> sequence motif

<220>
<221> VARIANT
<222> 1
<223> X is N or D

<220>
<221> VARIANT
<222> 2,4
<223> X is any amino acid

<220>
<221> MOD_RES
<222> 3
<223> PHOSPHORYLATION

<400> 15
Xaa Xaa Tyr Xaa Pro
1               5

<210> 16
<211> 5
<212> PRT
<213> Mus musculus

<220>
<223> and in Homo sapiens

<400> 16
Asp Gly Tyr Thr Pro
1               5

<210> 17
<211> 5
<212> PRT
<213> Mus musculus

<220>
<223> and in Homo sapiens

<400> 17
Asp Gly Tyr Met Pro
1               5

<210> 18
<211> 20
<212> DNA

<213> Artificial Sequence

<220>
<223> targeting Clec4n

<400> 18
ctgcccaaat cactggaagt                                                    20


<210> 19
<211> 20
<212> DNA
<213> Artificial Sequence

<220>
<223> targeting Clec4n

<400> 19
gaaattctgc tccgcttcag                                                    20


<210> 20
<211> 20
<212> DNA
<213> Artificial Sequence

<220>
<223> targeting Clec7a

<400> 20
gaaccacaag cccacagaat                                                    20


<210> 21
<211> 20
<212> DNA
<213> Artificial Sequence

<220>
<223> targeting Clec7a

<400> 21
taggaaggca aggctgagaa                                                    20


<210> 22
<211> 20
<212> DNA
<213> Artificial Sequence

<220>
<223> targeting Clec4d

<400> 22
tacgctggac gagaggaagt                                                    20


<210> 23
<211> 20
<212> DNA
<213> Artificial Sequence

<220>

<223> targeting Clec4d

<400> 23
acaggacagc aggtccaagt                                                    20


<210> 24
<211> 20
<212> DNA
<213> Artificial Sequence

<220>
<223> targeting Clec4e

<400> 24
caagtgctct cctggacgat                                                    20


<210> 25
<211> 20
<212> DNA
<213> Artificial Sequence

<220>
<223> targeting Clec4e

<400> 25
ctgtaagttc tgcccggaaa                                                    20


<210> 26
<211> 22
<212> DNA
<213> Artificial Sequence

<220>
<223> targeting TNF

<400> 26
caaaattcga gtgacaagcc tg                                                 22


<210> 27
<211> 18
<212> DNA
<213> Artificial Sequence

<220>
<223> targeting TNF

<400> 27
gagatccatg ccgttggc                                                      18


<210> 28
<211> 23
<212> DNA
<213> Artificial Sequence

<220>
<223> targeting IL-6

<400> 28

gaggatacca ctcccaacag acc                                                23

<210> 29
<211> 24
<212> DNA
<213> Artificial Sequence

<220>
<223> targeting IL-6

<400> 29
aagtgcatca tcgttgttca taca                                               24

<210> 30
<211> 20
<212> DNA
<213> Artificial Sequence

<220>
<223> targeting IL-12

<400> 30
ggaagcacgg cagcagaata                                                    20

<210> 31
<211> 24
<212> DNA
<213> Artificial Sequence

<220>
<223> targeting IL-12

<400> 31
aacttgaggg agaagtagga atgg                                               24

<210> 32
<211> 20
<212> DNA
<213> Artificial Sequence

<220>
<223> targeting IL-23

<400> 32
gcagattcca agcctcagtc                                                    20

<210> 33
<211> 20
<212> DNA
<213> Artificial Sequence

<220>
<223> targeting IL-23

<400> 33
ttcaacatat gcaggtccca                                                    20

<210> 34
<211> 25
<212> DNA
<213> Artificial Sequence

<220>
<223> targeting IL-1beta

<400> 34
caaccaacaa gtgatattct ccatg                                             25


<210> 35
<211> 21
<212> DNA
<213> Artificial Sequence

<220>
<223> targeting IL-1beta

<400> 35
gatccacact ctccagctgc a                                                 21


<210> 36
<211> 8
<212> PRT
<213> Artificial Sequence

<220>
<223> peptide

<400> 36
Phe Ala Gly Lys Gln Leu Glu Asp
1               5